# EUROPÄISCHE PATENTANMELDUNG

(11) **EP 2 169 576 A1**
(43) Veröffentlichungstag der Anmeldung: **31.03.2010**
(21) Anmeldenummer: 08165412.1
(22) Anmeldetag: 29.09.2008
(51) Int. Cl.: G06F 19/00

(54) **Verfahren zum Aktualisieren eines Zustands eines medizinisch verwendbaren Objekts**

(71) Anmelder: BrainLAB AG, 85622 Feldkirchen (DE)
(72) Erfinder: Urban, Alexander, 85570, Markt Schwaben (DE); Thiemann, Ingmar, 80686, München (DE)
(74) Vertreter: Schwabe - Sandmair - Marx

(57) **Zusammenfassung**

Die vorliegende Anmeldung betrifft ein medizindatentechnisches Verfahren zum Aktualisieren eines Zustands wenigstens eines Objekts, der eine medizinische Verwendbarkeit des wenigstens einen Objekts betrifft, das folgende Schritte umfasst: Bereitstellen von Objektdaten, die Lagedaten und den Zustand des wenigstens einen Objekts beschreiben; Bereitstellen von Änderungsdaten, die Änderungen des Zustands des wenigstens einen Objekts betreffen, **dadurch gekennzeichnet, dass** basierend auf einem Vergleich der Objektdaten mit den Änderungsdaten der Zustand des wenigstens einen Objekts aktualisiert wird.

## Beschreibung

Die vorliegende Erfindung betrifft ein medizindatentechnisches Verfahren zum Aktualisieren eines Zustands wenigstens eines medizinisch verwendbaren Objekts.

Bei praktischen Arbeiten im medizinischen Bereich muss meist darauf geachtet werden, dass die Arbeitsumgebung und die Arbeitsgeräte bestimmte Kriterien erfüllen. Hier kann es beispielsweise notwendig sein zu bestimmen, ob eine Arbeitsoberfläche, ein Raum, die Körperoberfläche oder Kleidung des medizinischen Personals, ein medizinisches Instrument, Körperteile des Patienten oder auch Substanzen, die in den Körper des Patienten eingebracht bzw. an ihm verwendet werden sollen, Anforderungen an Unversehrtheit, Reinheit und Sterilität erfüllen. Zudem ist es bei Operationen notwendig, sich nach dem Ende der Operation über den Verbleib chirurgischer Gerätschaften im Klaren zu sein, um zu verhindern, dass beispielsweise kein Instrument im Patientenkörper nach der Operation verbleibt und dort Schaden anrichtet. Außerdem ist es für die Verwaltung einer modernen medizinischen Einrichtung wichtig zu wissen, wie sie ihre Lagerhaltung führen muss, um wirtschaftlich zu arbeiten.

Gegenstand der vorliegenden Erfindung ist es, ein Verfahren bereitzustellen, mit dessen Hilfe der räumliche Aufenthaltsort bzw. die Bewegung eines Objekts bestimmt und/oder verfolgt werden kann, wobei das Objekt ein medizinisches Instrument, einen Körper oder Körperteil eines Mitglieds des medizinischen Personals und/oder eines Patienten, und/oder einen medizinisch verwendbaren Gegenstand umfassen kann. Der räumliche Aufenthaltsort eines Objekts kann durch die Lagedaten beschrieben werden, wobei die Lagedaten begrifflich den Ort des Objekts in einem Weltkoordinatensystem oder einem Koordinatensystem, das relativ zum Ort des Objekts definiert ist, beschreiben. Die Lagedaten können auch die Orientierung des Objekts in einem Weltkoordinatensystem oder einem Koordinatensystem, das relativ zum Ort des Objekts definiert ist, bzw. die Orientierung des Objekts relativ zu den Lagedaten eines anderen Objekts beschreiben, die in einem Weltkoordinatensystem oder einem Koordinatensystem, das relativ zum Ort des ersten Objekts definiert ist, angegeben werden. Die Lagedaten können auch einen Bewegungsvektor, der die Geschwindigkeit eines Objekts nach ihrem Betrag und ihrer Richtung bezeichnet, beinhalten. Der Begriff eines medizinisch verwendbaren Gegenstands kann ein medizinisches Instrument, ein Tupfer, eine Kompresse, Verbandmaterial, ein Schlauch, der zum Führen von Gas und/oder Flüssigkeit geeignet sein kann, ein Katheter, ein Ablagetisch, eine Patientenliege, ein Patientenbett und dergleichen mehr Gegenstände umfassen. Unter einem Objekt kann auch eine zweidimensional und/oder dreidimensional definierte räumliche Zone verstanden werden, die insbesondere einen medizinisch verwendbaren Gegenstand enthält und deren Rand insbesondere von dem Gegenstand (z.B. Patientenliege) beabstandet ist, um so z.B. einen Sicherheitsabstand zu sterilen Gegenständen zu gewährleisten. Gleichzeitig kann das erfindungsgemäße Verfahren das Objekt identifizieren, d.h. einer bestimmten Art bzw. Gattung von Objekten zuordnen (wie etwa die Zuordnung einer chirurgischen Schere zur Gattung der medizinischen Instrumente). Ferner ist das Verfahren dazu in der Lage, einen Zustand des Objekts zu erfassen, dem Objekt werden insofern Zustandsdaten zugeordnet. Dies beinhaltet beispielsweise die Feststellung, ob das Objekt rein oder unrein und/oder steril oder unsteril und/oder unbeschädigt oder beschädigt und/oder aktuell gebraucht (d.h. verwendet) wird oder ungebraucht (d.h. unverwendet) ist oder gebraucht (d.h. verwendet) worden ist. Auch kann der Zustand eine Beschreibung liefern, ob das Objekt ruhend oder in Bewegung ist. Einem Objekt kann auch eine Mehrzahl von Zuständen, d.h. insbesondere mehr als ein Zustand, also auch z.B. zwei oder drei oder mehr Zustände zugeordnet werden: Beispielsweise kann ein steriles Objekt aktuell gebraucht werden, weswegen ihm die beiden entsprechenden Zuständen "aktuell gebraucht" und "steril" zugeordnet werden können. Ferner soll das Verfahren die Verwaltung einer medizinischen Einrichtung in die Lage versetzen, ihre Lagerhaltung zu optimieren, indem über die Zuordnung von Zuständen zu einem Objekt, beispielsweise in einer Datenbank ein das Objekt betreffender Datensatz mit einer Information ausgestattet werden kann, die zum Beispiel angibt, ob das Objekt benutzt oder unbenutzt vorliegt. In einem solchen Fall beinhaltet das Objekt vorzugsweise einen medizinischen Gebrauchs- und/oder Verbrauchsgegenstand. Dann kann beispielsweise abgeleitet werden, ob eine Nachbestellung eines neuen gleichartigen Objekts beispielsweise beim Lieferanten notwendig ist. Ein Objekt kann auch eine räumliche Zone beinhalten, weshalb auch die Lagedaten von Zonen im Raum bestimmt und/oder verfolgt werden können. Diese Zonen können solche sein, die einen Raum und/oder eine Fläche bzw. Ebene, der bzw. die einen bestimmten Zustand besitzt, durch ihren Rand (Zonenrand) abgrenzen. Solch eine Zone kann ortsfest, aber auch beweglich definiert sein. Falls die Zone beweglich ist, also ihre Lagedaten ändern kann, so können ihre Lagedaten relativ zu Lagedaten eines anderen Objekts definiert sein. Für eine Zone sind im Rahmen dieser Erfindungen Zustandsänderungen möglich, wie sie für ein Objekt vorgestellt wurden. Eine Zone kann also auch beispielsweise mit einem Zustand "steril" ausgestattet sein, der auf den Zustand "unsteril" aktualisiert wird. Die Begriffe Änderung eines Zustand und Aktualisierung eines Zustands und Zuordnung eines Zustands werden im Rahmen dieser Erfindung gegeneinander austauschbar verwendet.

Die US 2007/01800810 zeigt ein System und Verfahren zur Radio Frequency Identification (RFID) und entsprechenden Kennzeichnung implantierbarer medizinischer Apparate. Diese Schrift offenbart ein System aus einem RFID-Sender/Empfänger und einem Transponder, das auch als Bestandteil eines Patientenmanagementsystems verwendet werden kann, um chronische Krankheitsverläufe zu beobachten.

Die WO 03001329 zeigt ein Datenverarbeitungssystem für medizinische Apparate, das Informationen über den medizinischen Apparat wie etwa eine Seriennummer oder Verwendungsdaten des medizinischen Apparates verwendet, Patienten- und Abrechnungsdaten verwalten kann sowie eine Schnittstelle zur Lagerhaltung und Wartungsdatenbank aufweist.

Die WO 2006045080 zeigt ein System zur Lagerhaltung im medizinischen Bereich, das unter anderem mit RFID-Technologie implementiert werden kann.

Die WO 200153919 zeigt ein System und ein Verfahren zur Verfolgung medizinischen Abfalls, das auf der Kennzeichnung von Transportmitteln beispielsweise mit optisch lesbaren Aufklebern beruht.

Aufgabe der vorliegenden Erfindung ist es, ein Verfahren zum Aktualisieren eines Zustands wenigstens eines Objekts, der eine medizinische Verwendbarkeit des wenigstens einen Objekts beschreibt, zur Verfügung zu stellen.

Gelöst wird diese Aufgabe durch die Gegenstände der unabhängigen Ansprüche. Die abhängigen Ansprüche betreffen vorteilhafte Ausführungsformen der Erfindung. Die Merkmale vorteilhafter Ausführungsformen können zwischen den verschiedenen Ausführungsformen kombiniert werden.

Vorteilhaft umfasst das erfindungsgemäße medizindatentechnische Verfahren zum Aktualisieren eines Zustands wenigstens eines Objekts, der eine medizinische Verwendbarkeit (Eigenschaft) des wenigstens einen Objekts betrifft und insbesondere beschreibt, folgende Schritte: Es werden Daten, die das wenigstens eine Objekt betreffen, bereitgestellt, wobei diese Objektdaten Lagedaten und den Zustand des wenigstens einen Objekts beschreiben.

Unter den Lagedaten des Objekts kann eine Beschreibung des Ortes bzw. der Lage des Objekts beispielsweise mithilfe von mehrdimensionalen Koordinaten (etwa kartesischen Koordinaten, Polarkoordinaten bzw. Kugelkoordinaten) und/oder Vektoren (die durch ihre Richtung bezüglich eines Referenzortes und einer Entfernung von diesem Referenzort den die Orientierung des Objekts kennzeichnen) verstanden werden. Die Lagedaten des Objekts können in einem Weltkoordinatensystem, aber auch relativ zu einem anderen Objekt, das sich von dem ersten Objekt unterscheiden kann, angegeben werden. Die Lagedaten werden beispielsweise erfasst, indem beispielsweise eine Kamera (also eine Detektionseinrichtung) mithilfe von elektromagnetischer Strahlung (insbesondere Licht im infraroten Spektralbereich), die von einer am Objekt angebrachten Markereinrichtung reflektiert oder ausgesendet wird, das Objekt erfasst und auf der Erfassung basierende Daten insbesondere an eine Datenverarbeitungsvorrichtung sendet, die dann die Koordinaten bzw. die Lagedaten des Objekts ermittelt. Es können aber auch Radio Frequency Identification Tags (RFIDs) am Objekt angebracht sein, so dass mit Hilfe eines RFID-Sensors bzw. von RFID-Sensoren die Lagedaten des Objekts über eine Datenverarbeitungsvorrichtung ermittelt werden können. Weitere Beispiele, wie z. B. optisches Erkennen der Form und Lage des Objekts, sind unten erwähnt.

Der Zustand des Objekts betrifft, insbesondere beschreibt beispielsweise dessen Verwendbarkeit (Eigenschaft), d.h. er umfasst Informationen beispielsweise bezüglich der Sterilität bzw. Unsterilität des Objekts, der Unversehrtheit oder Beschädigung (Verformung) des Objekts, der Reinheit bzw. Unreinheit des Objekts, der Verwendung bzw. Nichtverwendung des Objekts, z.B. ob es aktuell verwendet wird (d.h. z.B. in direktem und/oder indirektem beispielsweise physischen Kontakt mit einem Anwender steht bzw. der Anwender sich in der Zone befindet), verwendet worden ist oder noch nicht verwendet wurde. Verwendung bedeutet in diesem Zusammenhang wenigstens, dass das Objekt bzw. die Zone in einer Art und Weise angewandt bzw. benutzt wurde, in der die Anwendung bzw. Benutzung beabsichtigt war. Dadurch kann die Stellung des Objekts im Arbeitsablauf beschrieben werden und beispielsweise das Objekt ermittelt werden, das als nächstes im Arbeitsablauf zu verwenden ist. Es kann basierend auf dieser Ermittlung dem Anwender angezeigt werden, welches Objekt als nächstes zu verwenden ist. Die vorgenannten drei Verwendungszustände können insbesondere über die Bewegungshistorie des Objekts bestimmt werden. Wurde das Objekt (ab einem bestimmten Startzeitpunkt, der beispielsweise mit dem Beginn einer Operation übereinstimmt) noch nicht bewegt, so wird er als "noch nicht verwendet" bestimmt. Wurde er bereits bewegt, so wird als "bereits verwendet" bestimmt. Wird der Gegenstand aktuell bewegt oder innerhalb der jüngsten Zeit (z.B. innerhalb der letzten 10 Sekunden oder 60 Sekunden) so wird er als "aktuell verwendet" bestimmt. Die Bestimmung des Verwendungszustandes des Objekts kann alternativ oder zusätzlich auch auf anderen Bedingungen beruhen, insbesondere, ob das Objekt mit einem anderen Objekt in Kontakt gekommen ist oder nicht (siehe unten). Kommt ein Objekt, z.B. ein medizinisches Instrument, mit einem anderen Objekt (z.B. Arzt) in Kontakt, so kann darauf auf die aktuelle Verwendung des Objekts geschlossen werden. Diese Bestimmung als "aktuell verwendet" kann zusätzlich von der Bewegung des Objekts abhängig gemacht werden. Somit kann das erfindungsgemäße Verfahren oder System bestimmen, dass eine bestimmte Station (bestimmter Schritt) im Arbeitsablauf erreicht wurde, die mit der Verwendung dieses Instruments übereinstimmt. Entsprechend können dann Hinweisinformationen (z.B. Bildschirm und/oder Audio) an den Operateur ausgegeben werden, welcher Arbeitsschritt als nächster geplant war und/oder was für den aktuellen Schritt geplant ist. Dies kann insbesondere beim mehrschrittigen Registrieren von Gegenständen im Rahmen des IGS genutzt werden. Auch kann die Steuerung eines Gerätes, z.B. die Lichtsteuerung im Operationssaal auf der bestimmten Verwendung beruhen und insbesondere das aktuell verwendete Instrument und/oder das als nächstes zu verwendende Instrument (z.B. auf einem Tisch liegend) heller beleuchtet werden (als andere Objekte).

Die Erfindung erlaubt somit die Überwachung von Zustandsabläufen einer Vielzahl von Objekten. Beispielsweise kann ein Objekt 1 von einem Zustand noch "noch nicht verwendet" zu einem Zustand "verwendet" und schließlich in einen Zustand "bereits verwendet" übergehen. Danach kann ein weiteres Objekt 2 von einem Zustand "noch nicht verwendet" in den Zustand "aktuell verwendet" übergehen und nach einer Verwendungszeit in den Zustand "bereits verwendet" übergehen. Diese Zustandsabfolge kann für eine Vielzahl von Objekten bestimmt werden. Insbesondere kann diese Zustandabfolge auch für eine Vielzahl von Objekten geplant werden. Vorzugsweise werden deshalb Zustandsabfolgedaten bereitgestellt, die eine geplante Abfolge von Zuständen, z.B. für eine bestimmte Operation darstellen. Diese geplante Zustandsabfolge wird dann erfindungsgemäß mit den bestimmten Zuständen und somit mit der bestimmten Zustandsabfolge für eine Vielzahl von Objekten verglichen. Basierend auf dem Vergleich können insbesondere Hinweisinformationen ausgegeben werden. Beispielsweise kann der Arzt darauf hingewiesen werden, welches Objekt (körperliches Objekt, beispielsweise Instrument) als nächstes für die Verwendung geplant ist. Auch können Warnhinweise gegeben werden, falls ein anderes Objekt, als das geplante Objekt vom Arzt verwendet wird. Dadurch kann der Arzt beispielsweise darauf hingewiesen werden, falls er einen Instrumententyp (z.B. Scherentyp) verwendet, der mit dem geplanten Instrumententyp (geplanter Scherentyp) nicht übereinstimmt. Insbesondere kann auf diese Art und Weise auch überwacht werden, ob sämtliche körperlichen Objekte insbesondere am Ende einer Operation bzw. Behandlung (wie z.B. Tupfer aus dem Patientenkörper) entfernt wurden. Beispielsweise hat ein Tupfer den Zustand "aktuell verwendet" solange er sich noch in der Zone befindet, die den Patienten bzw. wenigstens ein Körperteil des Patienten (wie z.B. ein Organ und/oder ein Teil eines Organs) umgibt. Als geplante Zustandsabfolge kann vorgegeben sein, dass der Zustand des Tupfers von "aktuell verwendet" auf "bereits verwendet" übergeht, falls der Tupfer die Patientenzone verlassen hat. Als nicht mit der geplanten Zustandsabfolge vereinbar kann definiert sein, falls der Tupfer noch den Zustand "aktuell verwendet" hat, obwohl der Zustand der für das Zunähen geplanten Nadel von "noch nicht verwendet" auf "aktuell verwendet" übergeht. In diesem Fall wird dann eine Nichtübereinstimmung zwischen der geplanten Zustandsabfolge und der aktuellen bzw. bestimmten Zustandsabfolge vorteilhaft verschiedener Objekte (insbesondere körperlicher) festgestellt und ein Warnsignal kann ausgegeben werden. Das Warnsignal stellt ein Beispiel für eine Hinweisinformation dar, die basierend auf dem Vergleich der geplanten Zustandsabfolge mit der bestimmten Zustandsabfolgen vorteilhaft verschiedener Objekte ausgegeben wird.

Mit dem erfindungsgemäßen Verfahren können auch Objekte, insbesondere körperliche Objekte bilanziert werden. Das bedeutet, dass eine Anzahl von Objekten festgestellt und mit einer Vergleichsanzahl von Objekten verglichen werden kann, wobei auf diesem Vergleich basierend eine Information bzw. ein Hinweis an einen Benutzer ausgegeben werden kann. Insbesondere kann dem Zustand des Objekts eine Information hinzugefügt werden bzw. mit dem Zustand eine Information verknüpft werden, die die Anzahl von Objekten und/oder die Vergleichsanzahl von Objekten umfasst. Ferner kann dem Zustand eine Information hinzugefügt werden bzw. eine Information mit dem Zustand verknüpft werden, die Auskunft darüber gibt, ob das Objekt sich in einer bestimmten Zone (beispielsweise der Zone, in der der Ort des Objekts festgestellt wurde) aufhalten soll bzw. gerade nicht aufhalten soll. Außerdem kann dem Zustand eines Objekts eine Information hinzugefügt werden bzw. in eine Information mit dem Zustand verknüpft werden, die Auskunft darüber gibt, welche Art bzw. Gattung das Objekt ist und/oder welche bzw. wie viele andere Objekte anderer Art bzw. Gattung und/oder gleicher Art bzw. Gattung von Objekten sich insbesondere zum gleichen Zeitpunkt wie das betrachtete Objekt in einer bestimmten Zone aufhalten bzw. gerade nicht aufhalten sollen. Die Hinzufügung bzw. die Verknüpfung der Anzahl von Objekten zu dem bzw. mit dem Zustand des Objekts ist in Rahmen dieses Verfahrens möglich, muss aber nicht notwendigerweise stattfinden, um die Durchführbarkeit des Verfahrens zu gewährleisten. Beispielsweise kann zu Beginn einer Operation eine vorbestimmte Anzahl von körperlichen Objekten in einer Datenverarbeitungsvorrichtung abgespeichert sein. Diese Anzahl (Anfangsanzahl) kann vorteilhaft auch durch eine Eingabe des Benutzers über ein Eingabemodul der Datenverarbeitungsvorrichtung festgelegt werden. Ebenso kann der Benutzer den Beginn einer Operation der Datenverarbeitungsvorrichtung durch eine Eingabe anzeigen. Die Anfangsanzahl kann beispielsweise in den Änderungsdaten beinhaltet sein. Sodann können die körperlichen Objekte von der Detektionseinrichtung erfasst werden, wobei eine Anfangszahl der körperlichen Objekte ermittelt wird. Dabei können die körperlichen Objekte nach ihrer Anfangszahl pro Art bzw. Gattung eines körperlichen Objekts (z.B. pro Art einer bestimmten chirurgischen Schere) ermittelt werden, und diese Anfangszahl in einer Datenverarbeitungsvorrichtung abgelegt werden. Zu einem Zeitpunkt, der das Ende einer Operation anzeigt, wird dann wiederum eine Endanzahl der körperlichen Objekte (die auch jeweils getrennt nach Art bzw. Gattung der körperlichen Objekte aufgeschlüsselt werden kann) erfasst und in der Datenverarbeitungsvorrichtung abgespeichert. Ein Prozessor in der Datenverarbeitungsvorrichtung vergleicht sodann die Anfangsanzahl mit der Endanzahl. Die Endanzahl kann somit eine Vergleichsanzahl sein. Ergibt dieser Vergleich eine unerwünschte Abweichung der Endanzahl von der Anfangsanzahl, z.B. eine geringere Endanzahl als die Anfangsanzahl beträgt, so kann ein entsprechender Hinweis bzw. eine Information an den Benutzer ausgegeben werden. Es kann eine Information im Aktualisierungsschritt des Objekts in den Zustand eingetragen werden, die Auskunft über das Vergleichsergebnis gibt. Dieser Hinweis kann in visueller Form durch eine Anzeigeeinrichtung (z.B. einen Monitor) der Datenverarbeitungsvorrichtung erfolgen, aber auch eine Audioinformation (z.B. aus einem Lautsprecher) umfassen. Insbesondere kann der Benutzer darauf hingewiesen werden, dass zum Endzeitpunkt der Operation, d.h. zu dem Zeitpunkt, zu dem die Endanzahl ermittelt wurde, eine bestimmte Zahl körperlicher Objekte nicht erfasst werden konnte. Je nachdem, wo diese Objekte zuletzt im Rahmen des Verfahrens lokalisiert wurden, d.h. ihr Aufenthaltsort festgestellt wurde, kann eine visuelle Information bzw. Audioinformation an den Benutzer ausgegeben werden, wo sich das körperliche Objekt zuletzt befunden hat. Dies dient dazu, das Auffinden solcher nicht erfasster körperlicher Objekte für den Benutzer zu erleichtern. Zusätzlich kann an den Benutzer eine Information ausgegeben werden, wenn dieser letzte Aufenthaltsort des körperlichen Objekts sich in einer Zone befindet, die wenigstens einem Körperteil des Patienten (insbesondere dem Operationsfeld bzw. einer Patientenzone, also einer Zone, in der sich der Patient befindet) zu geordnet ist. Ein solcher Vergleich der Anzahl erfasster körperlicher Objekte mit einer Anfangsanzahl körperlicher Objekte kann auch zwischen Anfangszeitpunkt und Endzeitpunkt einer Operation durchgeführt werden, um beispielsweise zu verhindern, dass noch benötige Objekte sich nicht in Bereitschaft zur Benutzung (d.h. z.B. nicht in Reichweite des Benutzers) befinden. So kann sichergestellt werden, dass immer die benötigte Anzahl bestimmter körperlicher Objekte zur Verfügung steht. Eine solche zwischen Anfangszeitpunkt und Endzeitpunkt festgestellte Anzahl von körperlichen Objekten kann als Zwischenanzahl bezeichnet werden, wobei die Zwischenanzahl als Vergleichsanzahl verwendet werden kann.

Die Ermittlung der Anfangsanzahl bzw. Endanzahl kann auch zonenweise, d.h. pro Zone geschehen. Dabei wird die Anzahl körperlicher Objekte jeweils für eine bestimmte Zone ermittelt. Diese Ermittlung von Anfangsanzahl und Endanzahl kann wiederum zugleich nach Art bzw. Gattung der körperlichen Objekt aufgeschlüsselt werden. Somit kann sichergestellt werden, dass sich nur bestimmte Objekte in der betreffenden Zone befinden, deren Aufenthalt in dieser Zone auch erwünscht ist bzw. zu einem bestimmten Zeitpunkt im Verfahrensablauf erwünscht ist. Ebenso kann ermittelt werden, dass körperliche Objekte, die sich in der Zone aufhalten sollten, sich gerade dort nicht aufhalten. Diese Ermittlung von körperlichen Objekten pro Zone und ihres dort erwünschten / nicht erwünschten und tatsächlichen Aufenthaltes bzw. Nichtaufenthaltes in der Zone kann dadurch geschehen, dass die ermittelte Anzahl und Art von körperlichen Objekte in der jeweiligen Zone mit einer in der Datenverarbeitungsvorrichtung abgespeicherten Liste verglichen wird, die Informationen darüber umfasst, welche Objekte sich in welcher Anzahl in welcher Zone aufhalten sollen bzw. nicht sollen. Diese Liste kann beispielsweise in den Änderungsdaten beinhaltet sein. Diese Anforderung zum Aufenthalt bzw. Nicht-Aufenthalt eines körperlichen Objekts in einer bestimmten Zone kann abhängig vom Verfahrensablauf und/oder von wenigstens einem aus Anfangszeitpunkt, Zwischenzeitpunkt, Endzeitpunkt sein. Beispielsweise kann definiert sein, dass ein Tupfer zum Anfangszeitpunkt sich in einer Zone befinden soll, die einen Instrumententisch umschreibt, zum Endzeitpunkt sich aber beispielsweise immer noch auf dem Instrumententisch oder in einem Abwurfbehälter befinden soll. Ein dem Vergleich entsprechender Hinweis kann in visueller Form und/oder als Audioinformation an den Benutzer ausgegeben werden. Ebenso kann eine Information, die Auskunft über das Vergleichsergebnis gibt, im Aktualisierungsschritt für den Zustand des Objekts in den Zustand eingetragen werden. Dies kann z.B. über einen Monitor und/oder einen Lautsprecher, der an die Datenverarbeitungsvorrichtung angeschlossen ist, geschehen. Ein Beispiel für ein körperliches Objekt, das sich in einer bestimmten Zone aufhalten sollte, sind bestimmte Arten von Instrumenten in jeweils in einer bestimmten Anzahl auf einem Instrumententisch. Ein Beispiel für ein Objekt, das sich in einer bestimmten Zone nicht aufhalten soll, ist ein Tupfer, der bei dem definierten Endzeitpunkt der Operation sich noch in einer Zone befindet, die das Operationsfeld umschreibt bzw. beinhaltet. Dies kann beispielsweise bei bereits geschlossenen bzw. vernähtem Operationsfeld dadurch geschehen, dass das körperliche Objekt (der Tupfer) von der Kamera nicht visuell erfasst werden kann, der zuletzt festgestellte Ort des Tupfers jedoch in der betreffenden Zone liegt.

Ferner werden Änderungsdaten bereitgestellt, die Änderungen des Zustands des Objekts betreffen. Diese Änderungsdaten können Bedingungen beschreiben, unter denen sich der Zustand des wenigstens einen Objekts ändert. Diese Bedingungen können beispielsweise darauf beruhen, dass das Objekt den Zustand eines anderen Objekts annimmt, wenn das Objekt ein anderes Objekt berührt oder es sich ihm nähert bzw. wenn ein körperliches Objekt sich in einer Zone befindet bzw. sich dieser nähert bzw. wenn zwei Zonen einander berühren und/oder schneiden und/oder sich einander nähern. Unter der Annäherung zweier Zonen aneinander ist insbesondere zu verstehen, dass die äußeren Begrenzungen der Zonen, also die Ränder sich an wenigstens zwei Punkten (wobei jeweils ein Punkt zu einer der beiden Zonen gehört bzw. auf ihrem Rand liegt) räumlich nähern. Die Annäherung von zwei Zonen kann aber dadurch definiert sein, dass zwei Punkte, die im Inneren der beiden Zonen liegen (dass also jeweils einer der beiden Punkte zu einer der beiden Zonen gehört, aber nicht auf ihrem Rand liegt), sich einander räumlich nähern. Beispielsweise können dies im Falle von kugelförmig oder kreisförmig definierten Zonen deren Mittelpunkte sein, die sich in der Ebene und/oder im dreidimensionalen Raum räumlich nähern. Ein körperliches Objekt nähert sich dann einer Zone (die ein nicht-körperliches Objekt darstellt), wenn sein Ort sich entweder dem Rand der Zone oder einem (analog zur Annährung zweier Zonen aneinander definierten) Punkt im Inneren der Zone räumlich nähert. Auch diese Annäherung kann in zwei oder drei Dimensionen beschrieben werden. Ein körperliches Objekt besitzt Raumform, ist also insbesondere physisch greifbar. Insofern ist eine Zone nicht physisch greifbar (d.h. nicht körperlich), da sie virtuell definiert ist. Die oben beschriebene Annäherung von körperlichen Objekten und Zonen lassen sich wie folgt konkretisieren: Zwei Zonen können sich einander nähern, wenn beispielsweise um zwei Instrumententische jeweils relativ zu den Lagedaten des Instrumententisches eine Zone definiert ist. Werden etwa diese beiden Instrumententische zusammen geschoben (d.h. einander räumlich näher gebracht), so bewegen sich die zu ihnen gehörenden Zonen mit den Instrumententischen jeweils mit, was eine Annäherung der Zonen zur Folge hat. Ist einer der beiden Instrumententische mit einer Zone umgeben, die den Zustand "rein" besitzt, der andere mit einer Zone, die den Zustand "unrein" besitzt, so kann bei Überlappung der beiden Zonen infolge der Annäherung eine Änderung des Zustands "rein" in den Zustand "unrein" indiziert sein. Ein körperliches Objekt würde sich z.B. in der "unreinen" Zone befinden, wenn ein "steriles" Skalpell in dieser Zone läge, d.h. seine Lagedaten Koordinaten beschreiben, die sich mit den Koordinaten aus den Lagedaten der "unreinen" Zone decken bzw. von diesen räumlich umfasst werden. Dann wäre eine Zustandsänderung für das Skalpell von "steril" in "unsteril" indiziert. Auch kann zwischen den Zuständen "steril" und "unsteril" noch ein weiterer Zustand "Gefahr unsteril zu werden" genutzt werden. Dieser weitere Zustand kann z.B. dann als gegeben angenommen werden, wenn sich zwei Zonen (eine sterile und eine unsterile Zone) berühren. Dann kann ein entsprechendes Warnsignal ausgegeben werden, das auf dem Zustand "Gefahr unsteril zu werden" beruht. Die Zone kann beispielsweise erst dann als "unsteril" erkannt werden, wenn sich körperliche Objekte in der sterilen Zone und in der unsterilen Zone berühren.

Insbesondere können die Änderungsdaten auch beschreiben, welchen Zustand das körperliche Objekt bzw. die Zone annimmt bzw. beibehält, wenn die Bedingung zur Zustandsänderung erfüllt bzw. nicht erfüllt ist. Das Objekt, dessen Zustand beschrieben wird, befindet sich insbesondere in einem medizinischen Raum, in dem medizinische Hygienebedingungen vorgegeben sind, z.B. in einem Operationssaal, einem Wundnähraum, einem Untersuchungszimmer einem Krankenzimmer oder in einem Krankenkraftwagen. So kann beispielsweise eine Pinzette, die zuerst den Zustand "steril" besaß, den Zustand "unsteril" annehmen, wenn sie eine unsterile Umgebung bzw. Oberfläche berührt hat. Diese unsterile Oberfläche kann z.B. den Boden eines Operationssaales, ein anderes unsteriles medizinisches Instrument, ein unsteriles Kleidungsstück des medizinischen Personals und/oder eines Patienten, eine unsterile Flüssigkeit und/oder eine unsterile Ablagefläche beinhalten.

Die Bedingung zur Zustandsänderung des Objekts kann auch dergestalt sein, dass nach Ablauf einer bestimmten Zeitdauer das Objekt in den Zustand der Nichtverwendbarkeit versetzt wird. Darunter versteht man, dass das Objekt nicht mehr verwendet werden soll bzw. nicht mehr zur Verwendung vorgesehen ist, um beispielsweise die Arbeitsbedingungen (z.B. Anforderungen hinsichtlich Sterilität) einhalten zu können. Dies kann beispielsweise auf ein Medikament oder eine flüssigkeitsgetränkte Kompresse zutreffen, da diese der Atmosphäre nicht länger als eine bestimmte Zeitdauer ausgesetzt werden dürfen, da sie sonst beispielsweise aufgrund von Verdunstungsprozessen ihre Wirkstoffkonzentration und damit Nützlichkeit ändern. Die Bedingungen zur Zustandsänderung können auch eine Abfrage beinhalten, ob das Objekt Lagedaten besitzt, die einen Ort beschreiben, an dem es sich aus bestimmten Gründen nicht befinden darf bzw. gerade eben befinden soll. Basierend auf den Bedingungen, die in den Änderungsdaten beschrieben werden, kann eine Abfrage erfolgen, ob für das Objekt eine Bedingung zur Änderung eines Zustands gegeben ist. So muss beispielsweise die Pinzette ihren Zustand bzw. Anfangszustand "steril" nicht unbedingt ändern, wenn sie mit einem anderen Objekt in Berührung kommt, das bzw. die ebenfalls den Zustand "steril" besitzt. Eine Bedingung zur Zustandsänderung wäre in diesem Fall gegeben, wenn die sterile Pinzette ein anderes Objekt berühren würde, das den Zustand "unsteril" besitzt. Hier müsste die Pinzette ebenfalls den Zustand "unsteril" annehmen. Basierend auf dem Abgleich zwischen den Änderungsdaten und den Objektdaten wird also der Zustand des Objekts, der von den Objektdaten beschrieben wird, aktualisiert. Stellt sich bei dem Vergleich von Objektdaten mit Änderungsdaten also heraus, dass der Zustand des Objekts geändert werden soll, so ist eine Änderung des Zustands auf den Zustand, der aus den Änderungsdaten folgt, angezeigt; diese Änderung wird erfindungsgemäß durchgeführt. Aufgrund der vielfältigen Festlegung von Zuständen und deren Vergleichsmöglichkeiten miteinander, werden im Rahmen des vorliegenden Textes Benennungen von Zuständen rein exemplarisch verwendet. Eine andere Benennung bzw. Änderung in einen anders benannten Endzustand (d.h. den Zustand nach Änderung des Anfangszustands als Folge des Vergleichs) ist also nicht durch die Wortwahl ausgeschlossen.

Ferner können die Objektdaten so bereitgestellt werden, dass aus ihnen eine Bewegung des wenigstens einen Objekts nachvollzogen werden kann. Die Bewegung einer Zone umfasst auch eine teilweise Bewegung einer Zone, also z.B. eine Änderung der Form der Zone. Dies kann beispielsweise dadurch geschehen, dass die Objektdaten eine Zeitinformation, die zusätzlich zu den Lagedaten des Objekts aufgezeichnet werden, beinhalten. Dabei können die Zeitinformationen in einen Bezug zu den Lagedaten des Objekts gesetzt werden. Aus diesen Daten kann dann eine vergangene Bewegung des Objekts nachverfolgt, aufgezeichnet bzw. wiedergegeben werden. Insbesondere wird es also möglich, eine Reihenfolge der Lagedaten (also der Ortsinformationen) des Objekts festzulegen, die auf die tatsächliche zeitliche Abfolge der Lagedaten schließen lässt. Die Wiedergabe kann z.B. mit visuellen und/oder audiotechnischen Mitteln erfolgen, so kann beispielsweise auf einem Monitor die Bewegung des Objekts im Raum visuell dargestellt werden. Auch kann ein Audiosignal, das von einer an ein Zustandsaktualisierungssystem angeschlossenen Audiowiedergabeanlage ausgesendet wird, den Bewegungsablauf des Objekts beschreiben. Es ist außerdem Teil der Erfindung, dass aufgrund der Objektdaten eine zukünftige Bewegung des Objekts extrapoliert wird. So kann aus dem Bewegungsablauf der Vergangenheit bei beispielsweise linearer Extrapolation der Bewegung ermittelt werden, wohin sich das Objekt als nächstes bewegen wird. Dies kann dazu dienen, ein visuelles und/oder audiotechnisches Signal wie eben beschrieben auszugeben, das beispielsweise eine Warnung beinhaltet, sollte sich das Objekt in eine Richtung bewegen, die eine unerwünschte Zustandsänderung bzw. Zustandsaktualisierung des Objekts oder anderer Objekte und/oder eine Gefährdung von Personen und/oder Material zur Folge hätte. Letzteres träfe beispielsweise auf ein dem Operateur entglittenes steriles Skalpell zu, das in Richtung des Bodens eines Operationssaales fällt, wodurch es nicht nur unsteril werden könnte, sondern auch Körperteile des medizinischen Personals und/oder eines Patienten (wie etwa einen Fuß) der Gefahr einer Verletzung aussetzen würde.

Zudem können die Objektdaten zusätzlich zu den Lagedaten und Zustandsdaten des wenigstens einen Objekts Identifikationsdaten bezüglich des wenigstens einen Objekts umfassen. Diese Identifikationsdaten können beispielsweise analog zu den Lagedaten über eine Kamera erfasst werden. In diesem Fall kann insbesondere Form- bzw. Mustererkennung des Objekts oder auch die Verwendung von Oberflächen auf dem Objekt mit einem bestimmten, eindeutig dem Objekt bzw. seiner Art (Gattung) zuzuordnenden elektromagnetischen Reflexionskoeffizienten zur Identifikation des Objekts führen. Man kann auch Identifikationsdaten aus RFID-Informationen bzw. RFID-Tags, die an dem Objekt angebracht sind, mittels RFID-Sensoren und insbesondere einer Datenverarbeitungsvorrichtung auswerten, um das Objekt zu identifizieren. Diese Identifikationsdaten dienen dazu, das Objekt eindeutig zu identifizieren und/oder es einer Art bzw. Gattung zuzuordnen. So kann das Objekt, wenn es ein medizinisches Instrument darstellt (also körperlich ist), mit einer Seriennummer versehen sein, die beispielsweise in Form eines Schriftzuges und/oder eines Barcodes auf dem Objekt angebracht wird. Es kann aber auch ein Muster bzw. ein Schriftzug in die Oberfläche des Objekts eingeformt (beispielsweise graviert oder gestanzt) werden, um dann (z. B. optisch und/oder mechanisch) erfasst zu werden. Ein Vergleich mit einer zuvor bereitgestellten Datenbank kann dann zur Zuordnung dieser Seriennummer oder anderen Kennzeichnung zu einem bestimmten Datensatz verwendet werden, woraufhin das Objekt identifiziert und/oder einer Art bzw. Gattung zugeordnet werden kann. Für den Fall, dass das Objekt eine Zone umfasst, kann eine Markierung in Form der oben beschriebenen Seriennummer (z.B. auf einem Klebeschild oder einem direkt auf einer festen Oberfläche der Zone aufgebrachten, etwa eingravierten, Schriftzug) im Bereich dieser Zone angebracht werden, wobei dann für die Zone eine Identifikation nach weiteren Merkmalen möglich ist. Diese weiteren Merkmale können im Falle einer Zone insbesondere die Verträglichkeit von deren Oberflächen mit einem oder mehreren Reinigungsmitteln sein, so dass bei einer Zustandsänderung z.B. von "rein" auf "unrein" ein Vergleich mit den Identifikationsvergleichsdaten durchgeführt werden kann, um eine benötigte Menge an Reinigungsmitteln zur Wiederherstellung des Ursprungszustands "rein" zu berechnen. Die Identifikationsdaten können allerdings auch auf der Geometrie des Objekts basieren, indem gemäß einem Verfahren nach der EP 100 900 336 A1 die äußere Formgebung des Objekts in einem auf optischer Technologie aufbauenden Verfahren zur Identifikation des Objekts führt. Es können insbesondere Verfahren aus dem Bereich der Formerkennung und/oder Mustererkennung benutzt werden, um das Objekt zu identifizieren. Ebenso kann die Farbe des Objekts nach Erfassung durch eine Kamera und Auswertung eines auf dem erfassten Bild basierenden digitalen Datensatzes zur Identifikation des Objekts führen. Auch eine Oberflächeneigenschaft des Objekts kann zu diesem Zweck dienen, beispielsweise kann ein bestimmter elektromagnetischer Reflexionskoeffizient der Oberfläche des Objekts zu einer Identifikation bzw. Zuordnung des Objekt mithilfe beispielsweise eines spektroskopischen Verfahrens, das von der betreffenden Oberfläche reflektiertes Licht auswertet, dienen. Ferner kann auch eine Markervorrichtung, die an dem Objekt angebracht ist und aus Verfahren der navigationsgestützten Chirurgie bzw. Image Guided Surgery (IGS) bekannt ist, etwa über eine spezifische Anordnung der Markerelemente, deren Geometrie, Farbe bzw. Oberflächeneigenschaft zur Identifikation des Objekts bzw. der Zone herangezogen werden.

Basierend auf dem Vergleich der Objektdaten mit den Änderungsdaten kann eine Information über die Aktualisierung des Zustands des Objekts ausgegeben werden. Diese Information kann beispielsweise in visueller Form, d.h. in Bild- und/oder Textform auf einem Monitor ausgegeben werden. Vorteilhaft ist auch die Ausgabe eines Audiosignals, das eine derartige Information umfasst. Beispielsweise kann auch ein Vibrationssignal auf die Information hinweisen. Die Information kann auch in Form eines Signals bzw. einer Signalwelle an eine Datenverarbeitungsvorrichtung gesendet werden. Die Datenverarbeitungsvorrichtung kann dann beispielsweise die Information speichern und/oder für ihre Wiedergabe sorgen.

Die Objektdaten können auch auf Patientenanalysedaten basieren, die die Lage des wenigstens einen Objekts relativ zu einem Patientenkörper betreffen. Die Lagedaten des Objekts in einem Patientenkörper können somit mithilfe eines Patientenanalyseverfahrens, also eines Verfahrens, mit dessen Hilfe Informationen über die Geometrie eines Patientenkörpers gewonnen werden, ermittelt werden. Dies trifft insbesondere dann zu, wenn sich das Objekt wenigstens teilweise innerhalb eines Patientenkörpers befindet; es kann sich aber auch das gesamte Objekt innerhalb eines Patientenkörpers befinden. Insofern können die Objektdaten aus Patientenanalysedaten ermittelt werden bzw. auf ihnen basieren. Beispielsweise werden im Rahmen chirurgischer Navigationsverfahren Patientenanalysedaten als Modell des Patientenkörpers zur Verfügung gestellt. Dadurch kann zusammen mit den Lagedaten des Objekts eine Orientierung des Objekts bezüglich des Patientenkörpers bestimmt werden. Derartige Patientenanalysedaten werden für gewöhnlich mit bildgebenden Verfahren unter Verwendung von Röntgenstrahlung insbesondere mithilfe von computertomographischen und/oder magnetresonanztomographischen Daten bereitgestellt und als Navigationsdaten bzw. Image Guided Surgery (IGS)-Daten im Rahmen chirurgischer Navigationsverfahren verwendet. Zur Erfassung der Patientenanalysedaten kommen z.B. auch Ultraschallverfahren, herkömmliche Röntgenverfahren, Positronen-Emissions-Tomographieverfahren und Single Photon Emission Tomography-Verfahren in Frage.

Ferner können die Objektdaten Soll-Informationen und Ist-Informationen über die Geometrie des wenigstens einen Objekts beinhalten. Die Soll-Informationen können beispielsweise Informationen umfassen, die die Geometrie (Form) des Objekts beispielsweise eines medizinischen Instruments oder eines Implantats in einem Normzustand, d.h. z.B. einem zur Verwendung vorgesehenen Zustand wie einem unbeschädigten Zustand, beschreiben. Die Ist-Informationen können Informationen umfassen, wie sich die tatsächliche aktuelle Geometrie (Form) des Objekts darstellt. Aus einem Vergleich der Geometrieinformationen in den Soll-Informationen und Ist-Informationen kann dann beispielsweise auf eine Beschädigung (Verformung) eines körperlichen Objekts bzw. eine geometrische Veränderung der Zone geschlossen werden. Daraufhin kann dem Objekt ein bestimmter Zustand zugeordnet werden, beispielsweise im Falle einer Beschädigung also "beschädigt".

Der Zustand bzw. aktuelle Zustand des Objekts kann ferner beispielsweise in einer Datenbank mit einem Nominalzustand des Objekts verglichen werden. Dabei kann der Nominalzustand auf Informationen beruhen, die einen zuvor bekannten (z.B. vor Beginn der Operation erfassten) Aufenthaltsort und/oder den Verwendungszustand des Objekts umfassen. Nach Vergleichen von aktuellem Zustand und Nominalzustand des Objekts kann eine Information an eine Verwaltungseinheit gesendet werden um beispielsweise für eine Nachbestellung ähnlicher bzw. gleichartiger Objekte bei einem Lieferanten zu sorgen oder einen Reinigungsauftrag für die Zone zu erteilen. Lautet etwa der Nominalzustand des Objekts "unbenutzt", der aktuelle Zustand aber "benutzt", so macht es Sinn, an die Verwaltungseinheit eine Information zu senden, die auf die Notwendigkeit der Nachbestellung des betreffenden (körperlichen) Objekts bzw. einer Reinigung der Zone hinweist.

Die im erfindungsgemäßen Verfahren bereitgestellten Objektdaten, d.h. die Information über die Lage und den Zustand des Objekts können aufgezeichnet werden. Zusätzlich zu diesen Informationen können den Lagedaten und/oder dem Zustand zugehörige zeitliche Informationen gespeichert werden, um ein insbesondere vollständiges Protokoll der von dem wenigstens einem Objekt durchlaufenen Orte und/oder Zustände in Bezug auf ihre zeitliche Abfolge zu erhalten.

Abhängig von dem Vergleich der Objektdaten mit den Änderungsdaten bzw. abhängig von dem Ergebnis dieses Vergleiches kann von einer Steuereinrichtung ein Steuerimpuls ausgesendet werden, um weitere Schritte des Verfahrens auszulösen. Zum Beispiel kann mit dem erfindungsgemäßen Verfahren festgestellt werden, ob die Hand eines Operateurs ein Endoskop derart bedient, dass daraus offensichtlich wird, dass er es an einem Patienten anwenden möchte. Um für die Anwendung bessere Sichtverhältnisse zu schaffen, kann die Steuereinrichtung einen Steuerimpuls an eine Beleuchtungseinrichtung und/oder Verdunkelungseinrichtung senden, um die Lichtintensität im Arbeitsumfeld des Operateurs zu verringern, so dass er eine bessere Sicht durch die Optik des Endoskops erhält.

Das erfindungsgemäße Verfahren kann in Form eines Programms verfasst werden, das in einem permanenten Speichermedium gespeichert werden kann und von dort in einen Computer geladen werden kann, so dass es auf einem Computer abläuft und ihn veranlasst, die oben erwähnten Schritte bzw. Tätigkeiten auszuführen.

Bei einer Ausführungsform des erfindungsgemäßen Verfahrens wird eine Kameraanordnung, insbesondere mit mindestens zwei beabstandeten und auf das Objekt "blickenden" Kameras, verwendet, um ein Objekt bzw. eine Zone optisch (und räumlich) zu erfassen. Die zugrunde liegenden Prinzipien sind beispielsweise aus der bildgestützten Navigation (Image Guided Surgery) bekannt. Dies kann beispielsweise unter Verwendung von Infrarotstrahlung, die von einem Sender emittiert wird und von dem Objekt reflektiert wird und von der Kameraanordnung erfasst wird, erfolgen. Ebenso kann in gleicher Weise sichtbares Licht, insbesondere auch im Raum vorhandenes (z.B. nicht aus künstlichen Lichtquellen stammendes) Licht verwendet werden. Das Objekt kann dazu derart gestaltet sein, dass seine Oberfläche insbesondere Infrarotstrahlung und/oder sichtbares Licht in gewünschter Weise reflektiert, dazu können beispielsweise reflektierende Markierungen auf einem körperlichen Objekt bzw. in der Zone (und/oder an ihrer äußeren Begrenzung, d.h. am Rand der Zone) angebracht sein und/oder die Reflexionseigenschaft inhärent im Oberflächenmaterial des Objekts sein. Der Abstand oder die Position von der bzw. zur Kamera, etwa die Ausgangsposition bei Beginn der praktischen medizinischen Arbeit in einem Koordinatensystem, beispielsweise einem Weltkoordinatensystem oder relativ zur Kameraanordnung, kann bekannt sein oder kann in einem Kalibrierungsverfahren definiert bzw. ermittelt werden, so dass die Lagedaten des Objekts bzw. der Zone im Raum mittels der Kameraanordnung erfasst werden kann und/oder Veränderungen dieser Lage mittels der Kameraanordnung erfasst bzw. verfolgt werden können. Auch kann ein körperliches Objekt durch optische Erkennungsverfahren (z.B. Mustererkennungsverfahren) an seiner Form durch die Kameraanordnung erkannt werden und seine Lage mittels der Kameraanordnung bestimmt werden. Insbesondere wird zur Erkennung die detektierte Form mit abgespeicherten Formen der Objekte verglichen, so dass z.B. eine Schere eines bestimmten Typs an ihrer Form erkannt wird.

Für den Fall, dass die Erfassung des Objekts mit einer einzelnen Kamera erfolgt, werden zwei Raumkoordinaten (bzw. die räumliche Ausdehnung in zwei Dimensionen) des Objekts direkt ermittelt, die dritte Raumkoordinate (bzw. die räumliche Ausdehnung in der dritten Dimension) kann beispielsweise aus einer Größenänderung des Objekts im erfassten Bild berechnet werden. Dazu wird die erfasste Form des Objekts mit einer vorbekannten Form des Objekts in einer bekannten Position relativ zur Kamera verglichen; mittels elementarer geometrischer Überlegungen kann dann aus der abweichenden Form bzw. Größe des Objekts im erfassten Bild im Vergleich zu dem vorbekannten Bild der Abstand des Objekts relativ zur Kameraanordnung ermittelt werden.

In dem Fall, dass mehr als eine Kamera in der Kameraanordnung verwendet wird, ist vorzugsweise die Position der mindestens zwei Kameras der Kameraanordnung zueinander bekannt oder sie kann insbesondere in einem Kalibrierungsverfahren bestimmt werden. Ein solches Verfahren wird in der EP 100 681 028 A1 (Absätze [0007] bis [0008]) beschrieben und in die Offenbarung dieser Erfindung aufgenommen. Durch Verwendung von mehr als einer Kamera wird die Erfassung der Lagedaten des Objekts im Raum bzw. der Perspektive, mit der die Kameras das Objekt aufnehmen, möglich.

Die Kamera bzw. die Kameras der Kameraanordnung können insbesondere so ausgebildet sein, dass sie für Videoaufnahmen im Bereich des sichtbaren Lichts ausgebildet sind. So können sichtbares Licht emittierende und/oder reflektierende Objekte bzw. Oberflächen von Objekten aufgenommen werden. Die Kameraanordnung kann auch so konfiguriert sein, dass mit ihr sowohl Videoaufnahmen im Bereich des sichtbaren Lichts als auch im Infrarotbereich durchgeführt werden können, um insbesondere Infrarotstrahlung reflektierende und/oder emittierende Trackingmarkierungen (d.h. passive und/oder Markereinrichtungen), wie Marker oder Referenzsterne, erfassen zu können. Ferner kann die Kameraanordnung ortsfest oder beweglich angeordnet sein. Für den Fall, dass die Kameraanordnung beweglich ausgebildet ist, sollte nach jeder Ortsänderung der Kameraanordnung gegenüber der zuvor kalibrierten Position eine Neukalibrierung der Kameraposition vorgenommen werden.

Ferner wird in einer Datenspeichervorrichtung, die insbesondere einen permanenten Datenspeicher umfasst, ein Datensatz, der die Änderungsdaten betrifft, abgespeichert. In diesem Datensatz sind Koordinaten beinhaltet, die Bedingungen beschreiben, unter denen sich der Zustand des wenigstens einen Objekts ändert. Die Erfüllung dieser Bedingungen kann insbesondere darauf basieren, ob wenigstens zwei Objekte miteinander in insbesondere physischen Kontakt getreten sind. Die Erfüllung dieser Bedingungen kann auch darauf basieren, ob ein Objekt sich in einer Zone befindet und/oder ob zwei Zonen einander schneiden und/oder berühren. Zwei einander schneidenden Zonen können sich dergestalt schneiden, dass sie eine gemeinsame Schnittgerade bzw. Schnittstrecke für den Fall besitzen, dass wenigstens eine der beiden Zonen als Fläche oder Ebene, also zweidimensional (d.h. mit Raumkoordinaten, in denen wenigstens eine Koordinate für alle Punkte der Zone gleich ist) definiert ist. Falls beide Zonen als Raumzonen, d.h. dreidimensional definiert sind, ergibt ihr Schnitt wiederum eine Raumzone, d.h. eine Zone, die Grenzen besitzt, die mit Koordinaten in drei Dimensionen beschrieben werden können, wobei keine der Koordinaten fest sein muss. Ein Objekt befindet sich erfindungsgemäß in einer Zone, wenn die Zone zweidimensional definiert ist und der Ort des Objekts innerhalb der so definierten Fläche bzw. Ebene liegt. Im Falle einer dreidimensional definierten Zone liegt ein Objekt innerhalb der Zone, wenn der Ort des Objekts sich innerhalb bzw. auf der Grenze des so definierten Raumabschnitts befindet. Sollten die Grenzen mehrerer zweidimensional und/oder dreidimensional definierter Zonen aufeinander liegen, und sich ein Objekt auf einer solchen gemeinsamen Grenze befindet, so kann die Änderungsbedingung zweckmäßigerweise vorschreiben, dass ein bestimmter Zustand, der zu wenigstens einer der beiden Zonen gehört, dem Objekt zugeordnet werden kann. Befindet sich z.B. ein Objekt mit dem Zustand "steril" auf der gemeinsamen Grenze einer Zone mit dem Zustand "steril" und einer Zone mit dem Zustand "unsteril", so kann die Änderungsbedingung als Vorsichtsmaßnahme vorsehen, dass dem Objekt der Zustand "unsteril" zugeordnet wird. Die Koordinaten können als mehrdimensionale kartesische Koordinaten, Polarkoordinaten oder sphärische Koordinaten bzw. in Vektorform (d.h. beschrieben durch eine Richtungs- und Entfernungsangabe beispielsweise relativ zur Kameraanordnung) beschrieben sein. An die Kameraanordnung kann eine Datenverarbeitungsvorrichtung angeschlossen sein, die insbesondere einen permanenten Datenspeicher und einen Prozessor umfasst. Die Datenverarbeitungsvorrichtung führt den Vergleich zwischen den Objektdaten und den Änderungsdaten durch und kann darauf basierende Ergebnisse bzw. Informationen ausgeben. Diese Ergebnisse können insbesondere darauf hinweisen, dass der Zustand des Objekts geändert oder beibehalten wird. Die Datenverarbeitungsvorrichtung kann auch die Aufzeichnung bzw. Wiedergabe aufgezeichneter Lagedaten und/oder Zustandsdaten vollziehen. Im Datenspeicher können auch vorbekannte Daten über Objekte, wie deren Zustandsdaten und/oder Lagedaten abgespeichert werden und von der Datenverarbeitungsvorrichtung für den Vergleich gelesen werden. Solche vorbekannte bzw. vorgegebene Lagedaten können insbesondere Informationen darüber beinhalten, an welchen Orten sich ein körperliches Objekt nicht aufhalten soll. Über die Erfassung der Lagedaten und einen Vergleich mit den vorgegebenen Lagedaten kann insbesondere ermittelt werden, ob nach Abschluss einer Operation alle körperlichen Objekte (z.B. medizinische Instrumente) sich an einem zugewiesenen Ort befinden. Damit kann beispielsweise verhindert werden, dass bei Abschluss einer Operation Instrumente unbeabsichtigt im Patientenkörper verbleiben.
Figur 1 zeigt einen Instrumenttisch mit körperlichen Objekten und unterschiedlich definierten Zonen auf der Tischfläche.
Figur 2 zeigt eine Kameraanordnung, wie sie für gewöhnlich in chirurgischen Navigationsverfahren verwendet wird.
Figur 3 zeigt einen Instrumententisch mit körperlichen Objekten und unterschiedlich definierten Zonen auf der Tischfläche sowie einer individuellen Kameraanordnung.
Figur 4 zeigt eine mögliche Umgebung, in der ein medizinisch verwendbares, körperliches Objekt im Rahmen des erfindungsgemäßen Verfahrens angewandt wird.
Figur 5 zeigt einen Ablaufplan für das erfindungsgemäße Verfahren zur Zustandsänderung von Objekten.

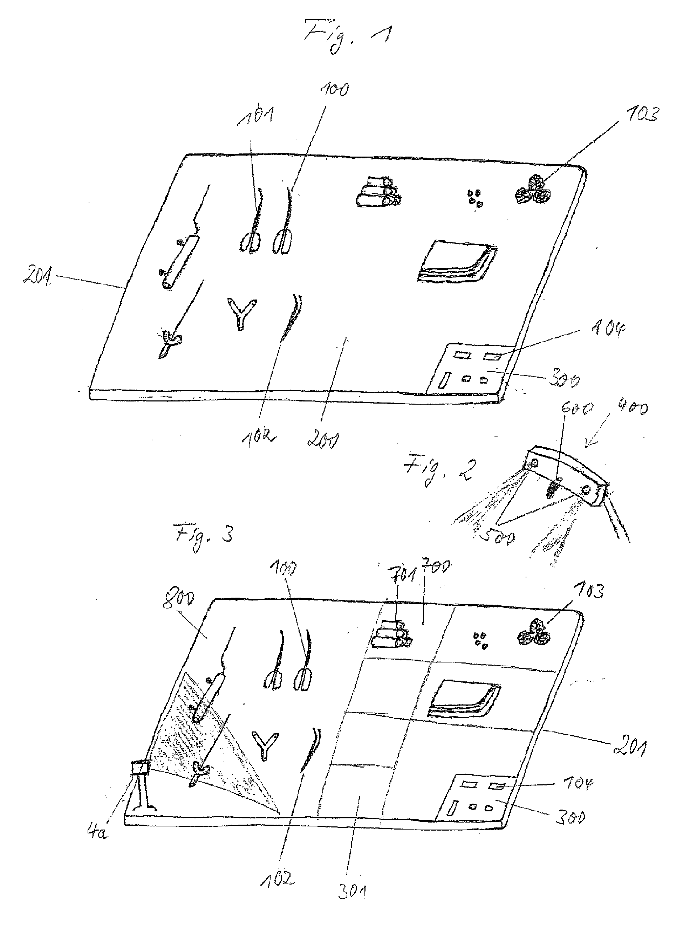

In Figur 1 ist ein Instrumententisch 201 dargestellt, auf dem sich unterschiedliche medizinische Objekte 100, 103 und 104 befinden. Die Objekte 100 und 103 befinden sich in einer Zone der Tischfläche des Instrumententisches 201, d.h. in einer Zone einer Oberfläche des Instrumententisches 201. Diese Zone ist eine Reinzone 200, d.h. eine Zone, die als insbesondere rein betrachtet werden kann. Das Objekt 104 befindet sich in einer Zone der Tischfläche, d.h. in einer Zone einer Oberfläche des Instrumententisches 201, die als Unreinzone 300 bezeichnet wird, d.h. als eine Zone, die als insbesondere unrein betrachtet werden kann. In der Unreinzone 300 können beispielsweise Objekte 100, 103 sowie das Objekt 104 abgelegt werden, wenn sie einen unsterilen Zustand bzw. einen Nichtverwendungszustand, d.h. einen Zustand, in welchem sie nicht zur Anwendung vorgesehen sind, besitzen bzw. angenommen haben.

Figur 2 zeigt eine Kameraanordnung 400, wie sie üblicherweise in Verfahren der Image Guided Surgery (IGS) bzw. in chirurgischen Navigationsverfahren verwendet wird. Die Kameraanordnung 400 beinhaltet einen Sender 600, der elektromagnetische Strahlung emittieren kann. Diese elektromagnetische Strahlung ist vorzugsweise Infrarotstrahlung und/oder sichtbares Licht. Die Kameraanordnung 400 beinhaltet ferner wenigstens eine, im dargestellten Fall zwei Empfänger bzw. Kameras 500, die in der Lage sind, vorzugsweise Infrarotstrahlung und/oder sichtbares Licht zu empfangen. Es können auch mehrere Sender 600 in der Kameraanordnung 400 angeordnet sein, z.B. ein Sender 600 zu jeder Kamera 500, wobei ein Sender 600 ringförmig um eine Linsenöffnung bzw. Empfängeröffnung der Kamera 500 angeordnet sein kann. Die Infrarotstrahlung bzw. das sichtbare Licht, die bzw. das von dem Sender 600 emittiert wird, kann beispielsweise von einer Oberfläche des Instrumententisches 201 bzw. eines Objekts 100, 103, 104 reflektiert werden. Die reflektierte elektromagnetische Strahlung kann dann in Kameras 500 aufgenommen werden, woraufhin ein entsprechendes Signal an eine Datenverarbeitungsvorrichtung bzw. einen Computer 1900 übermittelt werden kann. Aus diesem Signal kann die Datenverarbeitungsvorrichtung 1900 beispielsweise die Lagedaten der Oberfläche bzw. des Gegenstands berechnen, von dem die elektromagnetische Strahlung reflektiert wurde, also z.B. des Operationstisches 201, der Objekte 100, 103, 104 bzw. der Reinzone 200 und/oder der Unreinzone 300. Zu diesem Zweck können die Oberflächen des reflektierenden Gegenstands unterschiedlich ausgestaltet sein, um elektromagnetische Strahlung insbesondere im Infrarot- und/oder sichtbaren Bereich unterschiedlich reflektieren. Dadurch kann eine Identifizierung des Gegenstands, zu dem die reflektierende Oberfläche gehört, durch die Datenverarbeitungsvorrichtung 1900 ermöglicht werden. Dazu vergleicht die Datenverarbeitungsvorrichtung 1900 die Eigenschaften des Signals, das von der Kameraanordnung 400 entsprechend der aufgezeichneten elektromagnetischen Strahlung ausgesendet wird, mit zuvor gespeicherten Eigenschaften von den Oberflächen der Gegenstände 201, 100, 103, 104 reflektierte elektromagnetische Strahlung insbesondere im Infrarot- bzw. sichtbaren Bereich. Dadurch wird eine Identifizierung des reflektierenden Gegenstandes möglich. Diese Identifizierung kann individuell nach Gegenstand (z.B. kann zwischen zwei gleichartigen Scheren 100 und 101 unterschieden werden) oder nach Art bzw. Gattung des Gegenstandes (es kann unterschieden werden zwischen einer Schere 100 und einem Tupfer 103) erfolgen.

Figur 3 zeigt eine an einem Instrumententisch 201 befestigte (d.h. mit einem Instrumententisch 201 in physischem Kontakt stehende) individuelle Kameraanordnung 401. Auf dem Instrumententisch 201 sind Zonen seiner Oberfläche definiert, die beispielsweise die Reinzonen 700, 800 umfassen. Ferner ist eine Unreinzone 300 definiert. Einer Zone 301 ist beispielsweise kein Zustand zugeordnet. Der Anwender 1000 kann insbesondere dieser Zone 301 einen Zustand zuweisen, indem er eine entsprechende Eingabe an einer Eingabeeinrichtung 1904 einer Datenverarbeitungsvorrichtung 1900 vornimmt. Die individuelle Kameraanordnung 401 kann ausgebildet bzw. angeordnet sein, um nur eine der Zonen 300, 700, 800 mittels eines von ihr umfassten Senders 600 mit elektromagnetischer Strahlung zu bestrahlen und von der betreffenden Zone reflektierte elektromagnetische Strahlung zu empfangen. Eine solche Zone 300, 700, 800 kann insbesondere die Oberfläche einer Ablagemöglichkeit beinhalten. Die individuelle Kameraanordnung 401 entspricht in ihrem Aufbau der Kameraanordnung 400 aus Figur 2. Die individuelle Kameraanordnung 401 erfasst bzw. verfolgt somit innerhalb der von ihr betrachteten Zone 800 nur Gegenstände, die wie der Gegenstand 100 sich in einer zweidimensionalen Projektion der Zone 800 auf das Sichtfeld der Kameraanordnung 401 befindende Objekte 100. Falls die Kameraanordnung 401 ausgebildet ist, um nur die Reinzone 700 zu überwachen, erfasst bzw. verfolgt die Kameraanordnung 401 lediglich Gegenstände 701, die sich in einer zweidimensionalen Projektion der Reinzone 700 auf das Sichtfeld der Kameraanordnung 401. Analog erfasst die Kameraanordnung 401, wenn sie ausgebildet ist, um nur die Unreinzone 300 zu überwachen, lediglich Objekte 104, die sich einer zweidimensionalen Projektion der Unreinzone 300 auf das Sichtfeld der Kameraanordnung 401 befinden.

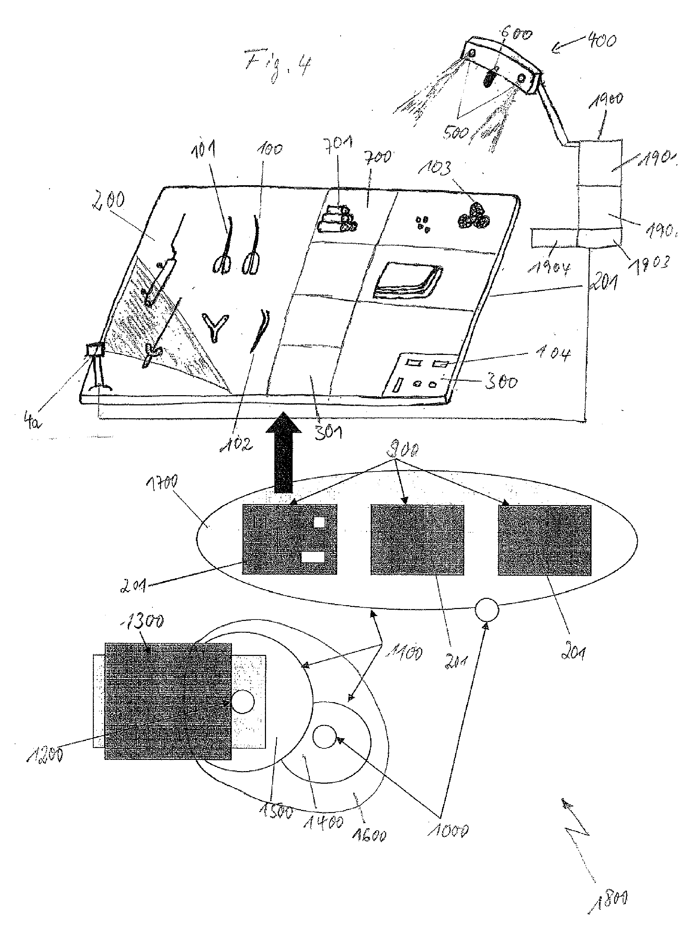

Figur 4 zeigt schematisch den Aufbau einer Arbeitsumgebung 1800, in dem das erfindungsgemäße Verfahren typischerweise angewandt wird. Dieser schematische Aufbau beinhaltet medizinisches Personal 1000, Zonen 1100, den Kopf bzw, einen Körperteil 1200 eines Patienten, Abdeckmaterial 1300 und Instrumententische 900. Jeder der dargestellten Instrumententische 900 gleicht in seinem grundlegenden Aufbau dem in Figur 3 bereits vorgestellten Instrumententisch 201. Um jeden in der Arbeitsumgebung 1800 vorhandenen Gegenstand können Zonen 1100 definiert werden, denen ein bestimmter Zustand (so z.B. "steril" bzw. "unsteril" und/oder "rein" bzw. "unrein" und/oder "verwendbar" bzw. "nicht verwendbar" und/oder "aktuell in Verwendung" bzw. "verwendet" bzw. "unverwendet" und/oder "ruhend" bzw. "in Bewegung") zugeordnet ist. Insbesondere kann dieser Zustand der Zonen 1100 identisch zum Zustand des Gegenstandes, relativ zu dem sie definiert ist, sein. Das bedeutet, dass alle Raumkoordinaten, die sich in den Zonen 1100 befinden, den jeweiligen Zustand der Zone 1100 zugeordnet bekommen. Die Zonen 1100 können sich zweidimensional und/oder dreidimensional erstrecken. Beispielhaft sind in Figur 4 derartige Zonen um den Körperteil 1200 eines Patienten (Zone 1500), den Standort von medizinischen Personal 1000 (Zone 1400)und um eine Vielzahl 900 von Instrumententischen 201 (Zone 1700) gezeigt. Auf jeden der Instrumententische 201 können wiederum Zonen 200, 300, 700 definiert werden. Dabei kann in der Ausführungsform von Figur 4 jede der Zonen 200, 300, 700 bzw. 1100 von einer eigens zu ihrer alleinigen Überwachung ausgebildeten Kameraanordnung 401 optisch erfasst werden. Es kann aber auch eine Mehrzahl von Zonen 200, 300, 700, 1100 bzw. alle Zonen 200, 300, 700, 1100 von Zonen von einer Kameraanordnung 400 optisch erfasst werden. Ferner können einzelne Zonen 1100 zu einer gemeinsamen Zone vereinigt werden. Dies kann wie im Falle der Zonen 1400 und 1500 mittels Definition einer Zone 1600, die die Zonen 1400 und 1500 umfasst, geschehen. Denkbar ist auch die Definition einer neuen Zone 1600 basierend auf eines Schnitts zwischen zwei dreidimensionalen Zonen 1400 und 1500. Im Fall zweidimensionaler Zonen 1400 und 1500 kann die Zone 1600 jedoch auch die Schnittmenge der zweidimensionalen Zonen 1400 und 1500 darstellen, wobei die Schnittmenge in diesem Fall als Schnittgerade (für den Fall, dass die Zonen 1400 und 1500 als Ebenen definiert sind) bzw. einer Schnittstrecke (für den Fall, dass die Zonen 1400 und 1500 als Flächen definiert sind) annehmen. Es kann ferner auch sein, dass zwei Zonen aus der Mehrzahl 1100 von Zonen 1500, 1600, 1700 sich durch Verschiebung eines Gegenstandes, um den sie definiert sein kann, derart verschiebt, dass sie in eine andere Zone eindringt. Dies wäre beispielsweise der Fall, wenn das medizinische Personal 1000, um das die Zone 1400 beispielsweise mit einem festen Radius um das medizinische Personal 1000 definiert ist, sich derart nah an den Körperteil 1200 des Patienten, um den mit einem festen Radius die Zone 1500 definiert ist, heranbewegt, dass die Zonen 1400 und 1500 einander scheiden würden. Sollte dies vermieden werden, da die Zone 1500 als beispielsweise rein und die Zone 1400 beispielsweise als unrein definiert ist, ist es erfindungsgemäß möglich, beispielsweise den Teil der Zone 1400, die sich mit der Zone 1500 schneidet, alleine als der Zone 1500 zugehörig zu definieren.

Die möglichen Bezugszeichen für ein Objekt 100, 101, 102, 103, 104, 701, 1000, 1200, 1300, 200, 300, 301, 700, 1400, 1500, 1600, 1700 werden im Folgenden als X abgekürzt, d.h. an Stelle von Objekt 100, 101, 102, 103, 104, 701, 1000, 1200, 1300, 200, 300, 301, 700, 1400, 1500, 1600,1700 wird Objekt X geschrieben.

Die möglichen Bezugszeichen für ein (körperliches) Objekt 100, 101, 102, 103, 104, 201, 701, 1200, 1300 werden im Folgenden als Y abgekürzt, d.h. an Stelle von Objekt 100, 101, 102, 103, 104, 201, 701, 1200, 1300 wird Objekt Y geschrieben.

Die möglichen Bezugszeichen für eine Zone 200, 300, 700, 1400, 1500, 1600, 1700 werden im Folgenden als Z abgekürzt, d.h. an Stelle von Zone 200, 300, 700, 1400, 1500, 1600, 1700 wird Zone Z geschrieben.

Unter den Lagedaten eines Objekts X kann eine Beschreibung des Ortes des Objekts X beispielsweise mithilfe von mehrdimensionalen Koordinaten (etwa kartesischen Koordinaten, Polarkoordinaten bzw. Kugelkoordinaten) und/oder Vektoren (die durch ihre Richtung bezüglich eines Referenzortes, beispielsweise des Ortes der Kameraanordnung 400, 401, und einer Entfernung von diesem Referenzort den Ort des Objekts X kennzeichnen) verstanden werden. Die Lagedaten des Objekts X können in einem Weltkoordinatensystem, aber auch relativ zu einem anderen Objekt X, das sich von dem ersten Objekt X unterscheiden kann, angegeben werden. Somit kann (beispielsweise in Vektorform) eine Orientierung des Objekts X, insbesondere relativ zu einem anderen Objekt X angegeben werden.

Erfindungsgemäß kann eine Anfangsanzahl von Objekten Y zu einem Anfangszeitpunkt einer Operation von einer Datenverarbeitungsvorrichtung 1901 und beispielsweise mithilfe der Kameraanordnung 400 ermittelt werden. Ebenso kann eine Endanzahl von Objekten Y zu einem Endzeitpunkt einer Operation ermittelt werden. Es ist auch möglich, eine Zwischenanzahl von Objekten Y zu einem Zeitpunkt, der zwischen Anfangszeitpunkt und Endzeitpunkt einer Operation liegt, zu ermitteln. Die Zwischenanzahl und/oder die Endanzahl von Objekten Y kann dann mit der Anfangsanzahl von Objekten Y verglichen werden, wobei basierend auf dem Ergebnis dieses Vergleichs ein Hinweis an einen Benutzer ausgegeben werden kann. Der Vergleich der Zwischenanzahl und/oder der Endanzahl mit der Anfangsanzahl kann durch einen Prozessor 1901 vorgenommen werden, wobei die zu vergleichenden Werte beispielsweise in einem Datenspeicher 1902 abgelegt bzw. gespeichert werden können. Der Anfangszeitpunkt und/oder der Endzeitpunkt der Operation und/oder die Anfangsanzahl und/oder die Zwischenanzahl und/oder die Endanzahl von Objekten Y kann automatisch von der Datenverarbeitungseinrichtung ermittelt werden oder auch durch manuelle Eingabe des Benutzers mittels einer Eingabeeinrichtung 1904 eingegeben werden. Die Hinweisinformation, die auf dem Vergleich der Anzahlen beruht und beispielsweise Informationen über ein unerwünschtes Abweichen der Anzahlen voneinander beinhalten kann, kann beispielsweise in einer Anzeigeeinrichtung 1903, die beispielsweise ein Monitor sein kann und/oder oder einen Lautsprecher beinhalten kann, in visueller Form und/oder als Audiosignal angezeigt werden. Die Anfangsanzahl kann für den gesamten Raum (z.B. OP-Saal) gelten und insbesondere der anfängliche Gesamtzahl der Objekte entsprechen. Sie kann aber auch für einzelne Zonen vorgegeben werden. Entsprechend wird die Zwischenzahl oder Endzahl für einzelne Zonen (z.B. Operationsfeld) bestimmt. Im Falle der Operationsfeld-Zone ist die Anfangsanzahl insbesondere Null.

Der Zustand des Objekts X betrifft, insbesondere beschreibt beispielsweise dessen Verwendbarkeit, d.h. er umfasst Informationen beispielsweise bezüglich der Sterilität bzw. Unsterilität des Objekts Y, der Unversehrtheit oder Beschädigung des Objekts X, der Reinheit bzw. Unreinheit des Objekts X, der Verwendung bzw. Nichtverwendung des Objekts X, z.B. ob es aktuell verwendet wird (d.h. z.B. im Falle eines körperlichen Objekts Y in direktem und/oder indirektem beispielsweise physischen Kontakt mit einem Anwender 1000 steht bzw. der Anwender 1000 sich in der Zone Z befindet), verwendet worden ist oder noch nicht verwendet wurde bzw. verwendet werden oder nicht verwendet werden kann. Verwendung bedeutet in diesem Zusammenhang wenigstens, dass das Objekt X in einer Art und Weise angewandt bzw. benutzt wird, in der die Anwendung bzw. Benutzung beabsichtigt ist. Dadurch kann die Stellung des Objekts X im Arbeitsablauf beschrieben werden und beispielsweise das Objekt X ermittelt werden, das als nächstes im Arbeitsablauf zu verwenden ist. Es kann basierend auf dieser Ermittlung dem Anwender 1000 angezeigt werden, welches Objekt X als nächstes bzw. als nächste zu verwenden ist. Ferner werden Änderungsdaten bereitgestellt, die Änderungen des Zustands des Objekts X betreffen. Diese Änderungsdaten können Bedingungen beschreiben, unter denen sich der Zustand des wenigstens einen Objekts X ändert. Diese Bedingungen können beispielsweise darauf beruhen, dass das Objekt Y den Zustand eines anderen Objekts Y annimmt, wenn es das andere Objekt Y berührt oder es sich ihm nähert bzw. wenn das Objekt Y sich in einer Zone Z befindet bzw. sich dieser nähert bzw. wenn zwei Zonen Z einander berühren und/oder schneiden und/oder sich einander nähern. Unter der Annäherung zweier Zonen Z aneinander ist insbesondere zu verstehen, dass die äußeren Begrenzungen der Zonen Z, also die Ränder sich an wenigstens zwei Punkten (wobei jeweils ein Punkt zu einer der beiden Zonen gehört bzw. Auf ihrem Rand liegt) räumlich nähern. Die Annäherung von zwei Zonen Z kann auch dadurch definiert sein, dass zwei Punkte, die im Inneren der beiden Zonen Z liegen (dass also jeweils einer der beiden Punkte zu einer der beiden Zonen Z gehört, aber nicht auf ihrem Rand liegt), sich einander räumlich nähern. Beispielsweise können dies im Falle von kugelförmig oder kreisförmig definiert Zonen Z deren Mittelpunkte sein, die sich in der Ebene und/oder im dreidimensionalen Raum räumlich nähern. Ein Objekt Y nähert sich dann einer Zone Z, wenn sein Ort sich entweder dem Rand der Zone Z oder einem (analog zur Annährung zweier Zonen Z aneinander definierten) Punkt im Inneren der Zone Z räumlich nähert. Auch diese Annäherung kann in zwei oder drei Dimensionen beschrieben werden. Insbesondere können die Änderungsdaten auch beschreiben, welchen Zustand das Objekt X annimmt, bzw. beibehält, wenn die Bedingung zur Zustandsänderung erfüllt bzw. nicht erfüllt ist. Das Objekt X, dessen Zustand beschrieben wird, befindet sich insbesondere in einem medizinischen Raum 1800, in dem medizinische Hygienebedingungen vorgegeben sind, z.B. in einem Operationssaal, einem Wundnähraum, einem Untersuchungszimmer einem Krankenzimmer oder in einem Krankenkraftwagen. So kann beispielsweise eine Pinzette 102, die zuerst den Zustand "steril" besaß, den Zustand "unsteril" annehmen, wenn sie eine unsterile Umgebung bzw. Oberfläche berührt hat. Diese unsterile Oberfläche kann z.B. den Boden eines Operationssaales und/oder ein anderes unsteriles medizinisches Instrument Y und/oder ein unsteriles Kleidungsstück des medizinischen Personals 1000 und/oder eines Patienten und/oder eine unsterile Flüssigkeit und/oder eine unsterile Ablagefläche 300 und/oder eine sterile oder unsterile Abdeckung 1300 und/oder einen unsterilen Körperteil 1200 eines Patienten und/oder eines Mitglieds des Personals 1000 beinhalten. Die Bedingung zur Zustandsänderung des Objekts X kann auch dergestalt sein, dass nach Ablauf einer bestimmten Zeitdauer das Objekt X in den Zustand der Nichtverwendbarkeit versetzt wird. Darunter versteht man, dass das Objekt X nicht mehr verwendet werden soll bzw. nicht mehr zur Verwendung vorgesehen ist, um beispielsweise die Arbeitsbedingungen (z.B. Anforderungen hinsichtlich Sterilität) einhalten zu können. Dies kann beispielsweise auf ein Medikament oder eine flüssigkeitsgetränkte Kompresse zutreffen, da diese der Atmosphäre nicht länger als eine bestimmte Zeitdauer ausgesetzt werden dürfen, da sie sonst beispielsweise aufgrund von Verdunstungsprozessen ihre Wirkstoffkonzentration und damit Nützlichkeit ändern. Die Bedingungen zur Zustandsänderung können auch eine Abfrage beinhalten, ob sich das Objekt Y an einem Ort befindet bzw. die Zone Z Koordinaten besitzt, an dem es bzw. an denen sie sich aus bestimmten Gründen nicht befinden darf bzw. gerade eben befinden soll. Basierend auf den Bedingungen, die in den Änderungsdaten beschrieben werden, kann eine Abfrage erfolgen, ob für das Objekt X eine Bedingung zur Änderung eines Zustands gegeben ist. So muss beispielsweise die Pinzette 102 ihren Zustand bzw. Anfangszustand "steril" nicht unbedingt ändern, wenn sie mit einem anderen Objekt X in Berührung kommt, das bzw. die ebenfalls den Zustand "steril" besitzt. Eine Bedingung zur Zustandsänderung wäre in diesem Fall gegeben, wenn die sterile Pinzette 102 ein anderes Objekt X berühren würde, das den Zustand "unsteril" besitzt. Hier müsste die Pinzette 102 ebenfalls den Zustand "unsteril" annehmen. Basierend auf dem Abgleich zwischen den Änderungsdaten und den Objektdaten wird also der Zustand des Objekts X der von den Objektdaten beschrieben wird, aktualisiert. Stellt sich bei dem Vergleich von Objektdaten mit Änderungsdaten also heraus, dass der Zustand des Objekts X geändert werden soll, so ist eine Änderung bzw. Aktualisierung des Zustands auf den Zustand, der aus den Änderungsdaten folgt, angezeigt; diese Änderung bzw. Aktualisierung wird erfindungsgemäß durchgeführt. Unter einem Objekt Y ist in diesem Zusammenhang auch eine Zone Z zu verstehen. Befindet sich z.B. ein Objekt mit dem Zustand "unrein" in einer Reinzone 200, 700, 1400, 1500, 1600, 1700 und wird dies im Rahmen des erfindungsgemäßen Verfahrens festgestellt, so kann der Zustand der Reinzone 200, 700, 1400, 1500, 1600, 1700 erfindungsgemäß auf "unrein" geändert bzw. aktualisiert werden, also aus einer Reinzone 200, 700, 1400, 1500, 1600, 1700 eine Unreinzone 200, 700, 1400, 1500, 1600, 1700 werden. Den Objekten Y kann dann auch der Zustand "unrein" zugeordnet werden bzw, ihr vorheriger Zustand auf den Zustand "unrein" geändert bzw. aktualisiert werden, wenn sie sich in der Reinzone 200, 700, 1400, 1500, 1600, 1700 zum Zeitpunkt der Änderung bzw. Aktualisierung ihres Zustands "rein" in bzw. auf unrein" befunden haben. Ebenso kann der Zustand "rein" einer Reinzone 200, 700, 1400, 1500, 1600, 1700 auf "unrein" geändert werden, wenn sie sich mit einer Unreinzone 300, 1400, 1500, 1600, 1700 schneidet. In diesem Fall wird der Zustand "rein" der Reinzone 200, 700, 1400, 1500, 1600, 1700 auf "unrein" geändert bzw. aktualisiert. Ebenso kann der Zustand einer Reinzone 200, 700, 1400, 1500, 1600, 1700, der der Zustand "rein" zugeordnet ist, auf "unrein" geändert werden, wenn ein Objekt Y sich in ihr befindet, das den Zustand "unrein" besitzt. Es kann aber auch nur der Zustand des Teils der Reinzone 200, 700, 1400, 1500, 1600, 1700 auf "unrein" geändert bzw. aktualisiert werden, der sich im Schnittbereich der Reinzone 200, 700, 1400, 1500, 1600, 1700 mit der Unreinzone 300, 1400, 1500, 1600, 1700 befindet. Dann kann z.B. der Teil der Reinzone 200, 700, 1400, 1500, 1600, 1700, der sich im Schnittbereich Reinzone 200, 700, 1400, 1500, 1600, 1700 mit der Unreinzone 300, 1400, 1500, 1600, 1700 befindet, als eine neue Unreinzone 300, 1400, 1500, 1600, 1700 definiert werden oder einer bestehenden Unreinzone 300, 1400, 1500, 1600, 1700 zugeordnet werden, d.h. er wird als Teil der bestehenden Unreinzone 300, 1400, 1500, 1600, 1700 bzw. zu dieser gehörend definiert.

Ferner können die Objektdaten so bereitgestellt werden, dass aus ihnen eine Bewegung des wenigstens einen Objekts Y, nachvollzogen werden kann. Dies kann beispielsweise dadurch geschehen, dass die Objektdaten eine Zeitinformation, die zusätzlich zu den Lagedaten des Objekts Y aufgezeichnet werden, beinhalten. Unter einem Objekt Y ist in diesem Zusammenhang auch eine Zone Z zu verstehen. Die Bewegung einer Zone Z umfasst auch eine teilweise Bewegung einer Zone Z, also z.B. eine Änderung der Form der Zone Z. Dabei können die Zeitinformationen in einen Bezug zu den Lagedaten des Objekts X gesetzt werden. Aus diesen Daten kann dann eine vergangene Bewegung des Objekts X nachverfolgt, aufgezeichnet bzw. wiedergegeben werden. Insbesondere wird es also möglich, eine Reihenfolge der Lagedaten bzw. Ortsinformationen des Objekts X festzulegen, die auf die tatsächliche zeitliche Abfolge der Lagedaten schließen lässt. Die Wiedergabe kann z.B. mit visuellen und/oder audiotechnischen Mitteln erfolgen, so kann beispielsweise auf einem Monitor die Bewegung des Objekts X im Raum visuell dargestellt werden. Auch kann ein Audiosignal, das von einer an ein Zustandsaktualisierungssystem angeschlossenen Audiowiedergabeanlage ausgesendet wird, den Bewegungsablauf des Objekts X beschreiben. Es ist außerdem Teil der Erfindung, dass aufgrund der Objektdaten eine zukünftige Bewegung des Objekts X extrapoliert wird. So kann aus dem Bewegungsablauf der Vergangenheit bei beispielsweise linearer Extrapolation der Bewegung ermittelt werden, wohin sich das Objekt X als nächstes bewegen wird. Dies kann dazu dienen, ein visuelles und/oder audiatechnisches Signal wie eben beschrieben auszugeben, das beispielsweise ein Warnung beinhaltet, sollte sich das Objekt X in eine Richtung bewegen, die eine unerwünschte Zustandsänderung bzw. Zustandsaktualisierung des Objekts X oder anderer Objekte X und/oder eine Gefährdung von Personen 1000 und/oder Material 1300 zur Folge hätte. Letzteres träfe beispielsweise auf ein dem Personal 1000 entglittenes steriles Skalpell zu, das in Richtung des Bodens eines Operationssaales fällt, wodurch es nicht nur unsteril werden könnte (d.h. sein Zustand beispielsweise von "steril" auf "unsteril" geändert bzw. aktualisiert werden würde), sondern auch Körperteile des medizinischen Personals 1000 und/oder eines Patienten (wie etwa einen Kopf 1200) der Gefahr einer Verletzung aussetzen würde.

Zudem können die Objektdaten zusätzlich zu den Lagedaten und Zustandsdaten des Objekts X Identifikationsdaten bezüglich des wenigstens einen Objekts X umfassen. Diese Identifikationsdaten dienen dazu, das Objekt X eindeutig zu identifizieren und/oder es einer Art bzw. Gattung zuzuordnen. So kann das Objekt, wenn es ein medizinisches Instrument 100, 101, 701, 103, 104, 1300 (also auch ein körperliches Objekt Y) darstellt, mit einer Seriennummer versehen sein, die beispielsweise in Form eines Schriftzuges und/oder eines Barcodes auf dem Objekt Y angebracht wird. Es kann aber auch ein Muster bzw. ein Schriftzug in die Oberfläche des Objekts Y eingeformt (beispielsweise graviert oder gestanzt) werden, um dann (z. B. optisch und/oder mechanisch) erfasst zu werden. Ein Vergleich mit einer zuvor bereitgestellten Datenbank, die Identifikationsvergleichsdaten beinhaltet, kann dann zur Zuordnung dieser Seriennummer oder anderen Kennzeichnung zu einem bestimmten Identifikationsvergleichsdatensatz verwendet werden, woraufhin das Objekt Y identifiziert und/oder einer Art bzw. Gattung zugeordnet werden kann. Für den Fall, das das Objekt X eine Zone Z (also ein nicht-körperliches Objekt) umfasst, kann eine Markierung in Form der oben beschriebenen Seriennummer (z.B. auf einem Klebeschild oder einem direkt auf einer festen Oberfläche der Zone aufgebrachten, etwa eingravierten, Schriftzug) im Bereich dieser Zone Z angebracht werden, wobei dann für die Zone Z eine Identifikation nach weiteren Merkmalen möglich ist. Diese weiteren Merkmale können im Falle einer Zone Z insbesondere die Verträglichkeit von deren Oberflächen mit einem oder mehreren Reinigungsmitteln sein, so dass bei einer Zustandsänderung z.B. von "rein" auf "unrein" ein Vergleich mit den Identifikationsvergleichsdaten durchgeführt werden kann, um eine benötigte Menge an Reinigungsmitteln zur Wiederherstellung des Ursprungszustands "rein" zu berechnen. Die Identifikationsdaten können allerdings auch auf der Geometrie des Objekts X basieren, indem gemäß einem Verfahren nach der EP 100 900 336 A1 die äußere Formgebung des Objekts X in einem auf optischer Technologie aufbauenden Verfahren zur Identifikation des Objekts X führt. Es können insbesondere Verfahren aus dem Bereich der Formerkennung und/oder Mustererkennung benutzt werden, um das Objekt X zu identifizieren. Ebenso kann die Farbe des Objekts Y nach Erfassung durch eine Kamera und Auswertung eines auf dem erfassten Bild basierenden digitalen Datensatzes zur Identifikation des Objekts X führen. Auch eine Oberflächeneigenschaft des Objekts 100, 101, 102, 103, 104, 701, 1300 kann zu diesem Zweck dienen, beispielsweise kann ein bestimmter elektromagnetischer Reflexionskoeffizient der Oberfläche des Objekts Y zu einer Identifikation bzw. Zuordnung des Objekt Y mithilfe beispielsweise eines spektroskopischen Verfahrens, das von der betreffenden Oberfläche reflektiertes Licht auswertet, dienen. Ferner kann auch eine Markervorrichtung, die an dem Objekt Y bzw. in der Zone Z angebracht ist und aus Verfahren der navigationsgestützten Chirurgie bzw. Image Guided Surgery (IGS) bekannt ist, etwa über eine spezifische Anordnung der Markerelemente, deren Geometrie, Farbe bzw. Oberflächeneigenschaft zur Identifikation des Objekts X herangezogen werden.

Basierend auf dem Vergleich der Objektdaten mit den Änderungsdaten kann eine Information über die Aktualisierung des Zustands des Objekts X ausgegeben werden. Diese Information kann beispielsweise in visueller Form, d.h. in Bild- und/oder Textform auf einem Monitor ausgegeben werden. Vorteilhaft ist auch die Ausgabe eines Audiosignals, das eine derartige Information umfasst. Beispielsweise kann auch ein Vibrationssignal, das von einer Vibrationseinrichtung erzeugt wird, auf die Information hinweisen. Eine solche Vibrationseinrichtung kann in z.B. physischem Kontakt mit wenigstens einem Mitglied des Personals 1000 stehen, so dass eine Vibration der Vibrationseinrichtung von diesem Wahrgenommen werden kann. Die Information kann auch in Form eines Signals bzw. einer Signalwelle an eine Datenverarbeitungsvorrichtung 1900 gesendet werden. Die Datenverarbeitungsvorrichtung 1900 kann dann beispielsweise die Information speichern und/oder für ihre Wiedergabe sorgen.

Die Objektdaten können auch auf Patientenanalysedaten basieren, die die Lage des wenigstens einen Objekts X relativ zu einem Patientenkörper betreffen. Die Lagedaten des Objekts X in einem Patientenkörper kann somit mithilfe eines Patientenanalyseverfahrens, also eines Verfahrens, mit dessen Hilfe Informationen über die Geometrie eines Patientenkörpers gewonnen werden, ermittelt werden. Dies trifft insbesondere dann zu, wenn sich das Objekt X wenigstens teilweise innerhalb eines Patientenkörpers befindet; es kann sich aber auch das gesamte Objekt X innerhalb eines Patientenkörpers befinden. Insofern können die Objektdaten aus Patientenanalysedaten ermittelt werden bzw. auf ihnen basieren. Beispielsweise werden im Rahmen chirurgischer Navigationsverfahren Patientenanalysedaten als Modell des Patientenkörpers bzw. eines Teils 1200 des Patientenkörpers zur Verfügung gestellt. Dadurch kann zusammen mit den Lagedaten des Objekts X eine Orientierung des Objekts X bezüglich des Patientenkörpers bestimmt werden. Derartige Patientenanalysedaten werden für gewöhnlich mit bildgebenden Verfahren unter Verwendung von Röntgenstrahlung insbesondere mithilfe von computertomographischen und/oder magnetresonanztomographischen Daten bereitgestellt und als Navigationsdaten bzw. Image Guided Surgery (IGS)-Daten im Rahmen chirurgischer Navigationsverfahren verwendet. Zur Erfassung der Patientenanalysedaten kommen z.B. auch Ultraschallverfahren, herkömmliche Röntgenverfahren, Positronen-Emissions-Tomographieverfahren und Single Photon Emission Tomography-Verfahren in Frage.

Ferner können die Objektdaten Soll-Informationen und Ist-Informationen über die Geometrie des wenigstens einen Objekts X beinhalten. Die Soll-Informationen können beispielsweise Informationen umfassen, die die Geometrie des Objekts Y, beispielsweise eines medizinischen Instruments Y (d.h. eines körperlichen Objekts) oder eines Implantats in einem Normzustand, d.h. z.B. einem zur Verwendung vorgesehenen Zustand wie einem unbeschädigten Zustand, beschreiben. Die Ist-Informationen können Informationen umfassen, wie sich die tatsächliche aktuelle Geometrie des Objekts X darstellt. Aus einem Vergleich der Geometrieinformationen in den Soll-Informationen und Ist-Informationen kann dann beispielsweise auf eine Beschädigung des Objekts Y bzw. eine geometrische Veränderung der Zone Z geschlossen werden. Daraufhin kann dem Objekt X ein bestimmter Zustand zugeordnet werden, beispielsweise im Falle einer Beschädigung also "beschädigt".

Der Zustand bzw. aktuelle Zustand des Objekts X kann ferner beispielsweise in einer Datenbank mit einem Nominalzustand des Objekts X verglichen werden. Dabei kann der Nominalzustand auf Informationen beruhen, die einen zuvor bekannten (z.B. vor Beginn der Operation erfassten) Aufenthaltsort und/oder den Verwendungszustand des Objekts X umfassen. Nach Vergleichen von aktuellem Zustand und Nominalzustand des Objekts Y, kann eine Information an eine Verwaltungseinheit gesendet werden um beispielsweise für eine Nachbestellung ähnlicher bzw. gleichartiger Objekte Y bei einem Lieferanten zu sorgen. Lautet etwa der Nominalzustand des Objekts "unbenutzt", der aktuelle Zustand aber "benutzt", so wird vorzugsweise an die Verwaltungseinheit eine Information gesendet, die auf die Notwendigkeit der Nachbestellung des betreffenden Objekts Y hinweist.

Die im erfindungsgemäßen Verfahren bereitgestellten Objektdaten, d.h. die Information über die Lage und den Zustand des Objekts X können aufgezeichnet werden. Zusätzlich zu diesen Informationen können den Lagedaten und/oder dem Zustand zugehörige zeitliche Informationen gespeichert werden, um ein insbesondere vollständiges Protokoll der von dem wenigstens einem Objekt X durchlaufenen Lagedaten und/oder Zustände in Bezug auf ihre zeitliche Abfolge zu erhalten. Insbesondere können die bestimmten Zeitdauern, die für die einzelnen Zustände bestimmt wurde und/oder die Zeiten der Zustandsänderungen genutzt werden, um diese mit den entsprechenden geplanten Werten zu vergleichen. Beispielsweise können für bestimmte Objekte minimale oder maximale Zeitdauern der Nutzung geplant werden und mit den aktuellen Nutzungsdauern verglichen werden. Überschreitet beispielsweise ein Tupfer die geplante Zeitdauer der Nutzung, so kann hierauf basierend eine Hinweisinformation ausgegeben werden, da der Tupfer möglicherweise (z.B. im Patientenkörper) vergessen wurde. Insbesondere kann diese Hinweisinformation (oder auch andere Hinweisinformation) eine Information darüber enthalten, in welcher Lage (also an welchem Ort) das Objekt von der Detektionseinrichtung (z.B. Kamera- und Navigatiorzssystem) zuletzt detektiert worden ist.

Abhängig von dem Vergleich der Objektdaten mit den Änderungsdaten bzw. abhängig von dem Ergebnis dieses Vergleiches kann von einer Steuereinrichtung (einem Prozessor 1901) ein Steuerimpuls ausgesendet werden, um weitere Schritte des Verfahrens auszulösen. Zum Beispiel kann mit dem erfindungsgemäßen Verfahren festgestellt werden, ob die Hand eines Operateurs 1000 ein Endoskop berührt oder derart bedient, dass daraus offensichtlich wird, dass er es an einem Patienten anwenden möchte. Um für die Anwendung bessere Sichtverhältnisse zu schaffen, könnte die Steuereinrichtung (einem Prozessor 1901) einen Steuerimpuls an Geräte des Behandlungsraumes, insbesondere an eine Beleuchtungseinrichtung und/oder Verdunkelungseinrichtung senden, um die Lichtintensität im Arbeitsumfeld 1800 des Operateurs 1000 zu verringern, so dass er eine bessere Sicht durch die Optik des Endoskops erhält.

Das erfindungsgemäße Verfahren kann in Form eines Programms verfasst werden, das in einem permanenten Speichermedium gespeichert werden kann und von dort in eine elektronische Datenverarbeitungsvorrichtung 1900 bzw. in einen Computer geladen werden kann, so dass es auf einem Computer abläuft und ihn veranlasst, die oben erwähnten Schritte bzw. Tätigkeiten auszuführen.

In dem erfindungsgemäßen Verfahren wird eine Kameraanordnung 400, 401, insbesondere mit mindestens zwei Kameras 500, verwendet, um ein Objekt X optisch zu erfassen. Dies kann beispielsweise unter Verwendung von Infrarotstrahlung, die von einem Sender 600 emittiert wird und von dem Objekt X reflektiert wird und von der Kameraanordnung 400, 401 erfasst wird, erfolgen. Ebenso kann in gleicher Weise sichtbares Licht, insbesondere auch im Raum vorhandenes (z.B. nicht aus künstlichen Lichtquellen stammendes) Licht verwendet werden. Das Objekt X kann dazu derart gestaltet sein, dass seine Oberfläche insbesondere Infrarotstrahlung und/oder sichtbares Licht in einer Weise reflektiert, dass die reflektierte elektromagnetische Strahlung bzw. die reflektierten elektromagnetischen Wellen von der Kamera 500 bzw. den Kameras 500 erfasst werden kann; dazu können beispielsweise reflektierende Markierungen auf dem Objekt Y bzw. in der Zone Z (und/oder an ihrer äußeren Begrenzung, d.h. am Rand der Zone Z) angebracht sein und/oder die Reflexionseigenschaft inhärent im Oberflächenmaterial des Objekts Y sein. Der Abstand oder die Position, etwa die Ausgangsposition bei Beginn der praktischen medizinischen Arbeit in einem Koordinatensystem, beispielsweise einem Weltkoordinatensystem oder relativ zur Kameraanordnung 400, 401, kann bekannt sein oder kann in einem Kalibrierungsverfahren definiert bzw. ermittelt werden, so dass die Lagedaten des Objekts X im Raum mittels der Kameraanordnung 400, 401 erfasst werden kann und/oder Veränderungen dieser Lage mittels der Kameraanordnung 400, 401 erfasst bzw. verfolgt werden können.

Für den Fall, dass die Erfassung des Objekts X mit einer einzelnen Kamera 500 erfolgt, werden zwei Raumkoordinaten des Objekts X direkt ermittelt, die dritte Raumkoordinate kann beispielsweise aus einer Größenänderung des Objekts X im erfassten Bild berechnet werden. Dazu wird die erfasste Form des Objekts X mit einer vorbekannten Form des Objekts X in einer bekannten Position relativ zur Kamera 500 verglichen; mittels elementarer geometrischer Überlegungen kann dann aus der abweichenden Form bzw. Größe des Objekts X im erfassten Bild im Vergleich zu dem vorbekannten Bild der Abstand des Objekts X relativ zur Kameraanordnung 400, 401 ermittelt werden.

In dem Fall, dass mehr als eine Kamera 500 in der Kameraanordnung 400, 401 verwendet wird, ist vorzugsweise die Position der mindestens zwei Kameras 500 der Kameraanordnung 400, 401 zueinander bekannt oder sie kann insbesondere in einem Kalibrierungsverfahren bestimmt werden. Ein solches Verfahren wird in der EP 100 681 028 A1 (Absätze [0007] bis [0008]) beschrieben und in die Offenbarung dieser Erfindung aufgenommen. Durch Verwendung von mehr als einer Kamera 500 wird die Erfassung der Lagedaten des Objekts X im Raum bzw. der Perspektive, mit der die Kameras 500 das Objekt X aufnehmen, möglich.

Die Kamera 500 bzw. die Kameras 500 der Kameraanordnung 400, 401 können insbesondere so ausgebildet sein, dass sie für Videoaufnahmen im Bereich des sichtbaren Lichts ausgebildet sind. So können sichtbares Licht emittierende und/oder reflektierende Objekte Y bzw. Oberflächen von Objekten Y bzw. Zonen Z aufgenommen werden. Die Kameraanordnung 400, 401 kann auch so konfiguriert sein, dass mit ihr sowohl Videoaufnahmen im Bereich des sichtbaren Lichts als auch im Infrarotbereich durchgeführt werden können, um insbesondere Infrarotstrahlung reflektierende und/oder emittierende Trackingmarkierungen (d.h. passive und/oder Markereinrichtungen), wie Marker oder Referenzsterne, erfassen zu können. Ferner kann die Kameraanordnung 400, 401 ortsfest oder beweglich angeordnet sein. Für den Fall, dass die Kameraanordnung 400, 401 beweglich ausgebildet ist, sollte nach jeder Ortsänderung der Kameraanordnung 400, 401 gegenüber der zuvor kalibrierten Position eine Neukalibrierung der Kameraposition 400, 401 vorgenommen werden.

Ferner wird in einer Datenspeichervorrichtung, die insbesondere einen permanenten Datenspeicher bzw. ein permanentes Speichermedium 1902 umfasst, ein Datensatz, der die Änderungsdaten betrifft, abgespeichert. In diesem Datensatz sind Koordinaten beinhaltet, die Bedingungen beschreiben, unter denen sich der Zustand des wenigstens einen Objekts Y bzw. der wenigstens einen Zone Z ändert. Die Erfüllung dieser Bedingungen kann insbesondere darauf basieren, ob wenigstens zwei Objekte Y miteinander in insbesondere physischen Kontakt getreten sind. Die Erfüllung dieser Bedingungen kann auch darauf basieren, ob ein Objekt Y sich in einer Zone Z befindet und/oder ob zwei Zonen Z einander schneiden. Zwei einander schneidende Zonen Z können sich dergestalt schneiden, dass sie eine gemeinsame Schnittgerade bzw. Schnittstrecke für den Fall besitzen, dass wenigstens eine der beiden Zonen als Fläche oder Ebene, also zweidimensional (d.h. mit Raumkoordinaten, in denen wenigstens eine Koordinate für alle Punkte der Zone gleich ist) definiert ist. Falls beide Zonen Z als Raumzonen, d.h. dreidimensional definiert sind, ergibt ihr Schnitt wiederum eine Raumzone, d.h. eine Zone Z, die Grenzen besitzt, die mit Koordinaten in drei Dimensionen beschrieben werden können, wobei keine der Koordinaten fest sein muss. Ein körperliches Objekt Y befindet sich erfindungsgemäß in einer Zone Z, wenn die Zone Z zweidimensional definiert ist und der Ort des Objekts Y innerhalb der so definierten Fläche bzw. Ebene Z liegt. Im Falle einer dreidimensional definierten Zone Z liegt ein Objekt Y innerhalb der Zone Z, wenn der Ort des Objekts Y sich innerhalb bzw. auf der Grenze des so definierten Raumabschnitts Z befindet. Sollten die Grenzen mehrerer zweidimensional und/oder dreidimensional definierter Zonen Z aufeinander liegen und sich ein körperliches Objekt Y sich auf einer solchen gemeinsamen Grenze befinden, so kann die Änderungsbedingung zweckmäßigerweise vorschreiben, dass ein bestimmter Zustand, der zu wenigstens einer der beiden Zonen Z gehört, dem Objekt Y zugeordnet werden kann. Befindet sich z.B. ein Objekt Y mit dem Zustand "steril" auf der gemeinsamen Grenze einer Zone Z mit dem Zustand "steril" und einer Zone Z mit dem Zustand "unsteril", so kann die Änderungsbedingung als Vorsichtsmaßnahme vorsehen, dass dem Objekt Y der Zustand "unsteril" zugeordnet wird. Die Koordinaten können als mehrdimensionale kartesische Koordinaten, Polarkoordinaten oder sphärische Koordinaten bzw. in Vektorform (d.h. beschrieben durch eine Richtungs- und Entfernungsangabe beispielsweise relativ zur Kameraanordnung 400, 401) beschrieben sein. An die Kameraanordnung 400, 401 kann eine Datenverarbeitungsvorrichtung 1900 angeschlossen sein, die insbesondere einen permanenten Datenspeicher 1902 und einen Prozessor 1901 umfasst. Die Datenverarbeitungsvorrichtung 1900 führt den Vergleich zwischen den Objektdaten und den Änderungsdaten durch und kann darauf basierende Ergebnisse bzw. Informationen ausgeben. Die Datenverarbeitungsvorrichtung 1900 kann auch die Aufzeichnung bzw. Wiedergabe aufgezeichneter Lagedaten und/oder Zustandsdaten vollziehen. Im Datenspeicher 1902 können auch vorbekannte Daten über Objekte X, wie deren Zustandsdaten und/oder Lagedaten abgespeichert werden und von der Datenverarbeitungsvorrichtung 1900 für den Vergleich gelesen werden. Solche vorbekannten bzw. vorgegebenen Lagedaten können insbesondere Informationen darüber beinhalten, an welchen Orten sich ein körperliches Objekt Y nicht aufhalten soll. Über die Erfassung der Lagedaten und einen Vergleich mit den vorgegebenen Lagedaten kann insbesondere ermittelt werden, ob nach Abschluss einer Operation alle körperlichen Objekte (z.B. medizinische Instrumente) Y sich an einem zugewiesenen Ort befinden. Damit kann beispielsweise verhindert werden, dass bei Abschluss einer Operation Instrumente unbeabsichtigt im Patientenkörper verbleiben.

Die geplante Abfolge von Zuständen für verschiedene Objekte kann insbesondere eine Anfangslage mit einem Anfangszustand und eine Endlage mit einem Endzustand umfassen. Beispielsweise kann für jeden Gegenstand, insbesondere für jedes Instrument dies definiert werden. So kann beispielsweise die Anfangslage einer Schere auf einem Tisch sein, wie er in Figur 1 gezeigt ist. Der Anfangszustand ist "noch nicht verwendet". Der geplante Endzustand ist "bereits verwendet" und die Endlage kann beispielsweise auf einem anderen Tisch sein, auf dem nicht mehr sterile Instrumente abgelegt werden. Entsprechend können beispielsweise im Falle von einmalig gebrauchten Gegenständen die Lage von Abfallbehältern als Endlage für diese einmal gebrauchten Gegenstände (Objekte) definiert werden. Insbesondere kann bestimmt werden, dass der Gegenstand die geplante Endlage und den geplanten Endzustand erreicht hat, wenn er in einer Zone um den Abfallbehälter herum eingetreten ist. Insbesondere können zu diesem Zweck auch optische Detektoren und Kameras an den Abfallbehältern angebracht sein. Die Detektionseinrichtung, die diese Kameras umfasst, kann dann anhand der Form und gegebenenfalls anhand der Farbe die gebrauchten Gegenstände erkennen und ihre Lage als innerhalb der "Abfall-Zone" befindlich erkennen.

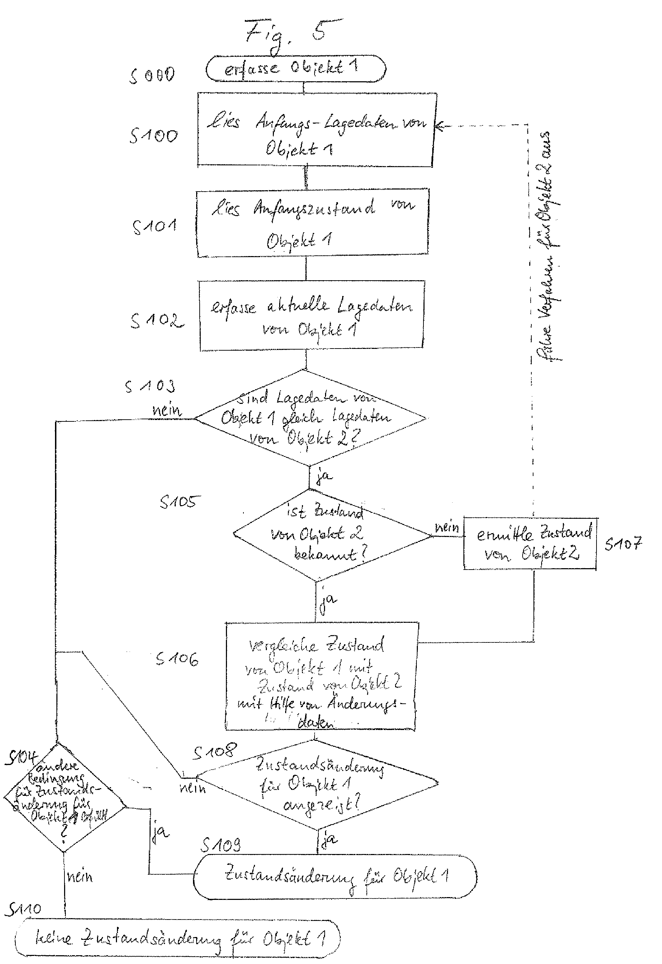

Figur 5 fasst in einem Ablaufplan das oben geschilderte Verfahren, in dem ermittelt wird, ob für ein Objekt eine Bedingung zur Zustandsänderung erfüllt ist, zusammen. Schritt S000 beinhaltet die optische Erfassung eines Objekts X durch die Kameraeinrichtung 400, 401. In Schritt S100 liest der Prozessor 1901 aus dem Datenspeicher 1902 die vorbekannten (z.B. zu Beginn der Operation festgelegten) Lagedaten eines Objekts X ein. In Schritt S101 liest der Prozessor 1901 den vorbekannten Anfangszustand des Objekts X ein. Anschließend werden in Schritt S102 vom Prozessor 1901 die aktuellen Lagedaten des Objekts X mittels der Kameraeinrichtung 400, 401 eingelesen und vorteilhaft zur weiteren Verwendung im Datenspeicher 1902 gespeichert.

In Schritt S103 erfolgt eine Abfrage, ob die Ortsinformationen in den Lagedaten des Objekts X gleich den Ortsinformationen in den Lagedaten eines anderen Objekts X sind bzw. von diesen umfasst werden. Die Lagedaten des anderen Objekts X werden entweder in diesem Schritt S103 mittels der Kameraeinrichtung 400, 401 erfasst oder sind vorbekannt und werden vom Prozessor 1901 vom Datenspeicher 1902 eingelesen.

Wenn Schritt S103 ergibt, dass die Lagedaten des Objekts X gleich den Lagedaten eines anderen Objekts X sind bzw. von letzteren umfasst werden, erfolgt in Schritt S105 eine Abfrage, ob der Zustand des anderen Objekts X bekannt ist. Wenn dieser Zustand nicht (z.B. aus einer im Datenspeicher 1902 gespeicherten Datenbank) bekannt ist, wird er in Schritt S107 ermittelt. Dies kann z.B. über den Eintritt in Schritt S100 für die Ermittlung des Zustands des anderen Objekts X erfolgen.

Wenn der Zustand des anderen Objekts X dann ermittelt ist bzw. er in Schritt S105 als bekannt ermittelt wurde, wird in Schritt S106 der Zustand des Objekts X mit dem Zustand des anderen Objekts verglichen anhand vorhandener Änderungsbedingungen aus den Änderungsdaten. Das heißt, es wird überprüft, ob für einen Zustand des Objekts X ein entsprechender bzw. entgegen gesetzter Zustand des anderen Objekts X vorliegt und ob für diese Kombination von Zuständen in den Änderungsdaten eine Änderungsbedingung vorhanden ist. Mithilfe einer Abfrage in S108 aus der Datenbank im Datenspeicher 1902, in der die Änderungsdaten gespeichert sind, wird ermittelt, ob eine Bedingung für eine Zustandsänderung des Objekts X erfüllt ist.

Wenn diese Änderungsbedingung erfüllt ist, wird in Schritt S109 der Zustand des Objekts entsprechend der Anweisung aus der Änderungsbedingung geändert. Der neue Zustand des Objekts X kann dann im Datenspeicher 1902 gespeichert werden.

Ist keine Bedingung für eine Zustandsänderung des Objekts X erfüllt, erfolgt in Schritt S104 eine Abfrage aus der auf dem Datenspeicher 1902 gespeicherten Datenbank der Änderungsdaten, ob eine andere Änderungsbedingung für eine Zustandsänderung des Objekts X erfüllt ist. Diese andere Änderungsbedingung kann z.B. darauf abzielen, ob das Objekt X gegenüber den vorbekannten Lagedaten bewegt wurde. Ist keine weitere Änderungsbedingung erfüllt, endet das Verfahren in Schritt S110 ohne Zustandsänderung für das Objekt X, d.h. der aktualisierte Zustand des Objekts X ist gleich dem Anfangszustand des Objekts X. Ist aber eine andere Bedingung zur Zustandsänderung erfüllt, wird in Schritt S109 der Zustand des Objekts X entsprechend der Änderungsbedingung geändert bzw. auf den entsprechenden Zustand aktualisiert. Der neue Zustand des Objekts X kann dann im Datenspeicher 1902 gespeichert werden.

Ergibt sich in Schritt S103, dass die Lagedaten des Objekts X nicht die gleiche Ortsinformation wie die Lagedaten eines anderen Objekts X sind bzw. nicht von letzteren umfasst werden, so erfolgt in Schritt S104 eine Abfrage aus der auf dem Datenspeicher 1902 gespeicherten Datenbank der Änderungsdaten, ob eine andere Änderungsbedingung für eine Zustandsänderung des Objekts X erfüllt ist. Diese andere Änderungsbedingung kann z.B. darauf abzielen, ob das Objekt X gegenüber den vorbekannten Lagedaten bewegt wurde. Ist keine weitere Änderungsbedingung erfüllt, endet das Verfahren in Schritt S 110 ohne Zustandsänderung für das Objekt X, d.h. der aktualisierte Zustand des Objekts X ist gleich dem Anfangszustand des Objekts X. Ist aber eine andere Bedingung zur Zustandsänderung erfüllt, wird in Schritt S109 der Zustand des Objekts X entsprechend der Änderungsbedingung geändert bzw. auf den entsprechenden Zustand aktualisiert. Der neue Zustand des Objekts X kann dann im Datenspeicher 1902 gespeichert werden.

Die Schritte S104, S106 und S108 können für ein Objekt X, dem mehrere Zustände zugeordnet sind, für jeden einzelnen dieser Zustände durchgeführt werden. Dabei kann für jeden der Zustände die Erfüllung einer Änderungsbedingung überprüft werden bzw. der Vergleich mit einem entsprechenden (z.B. gleichen oder entgegengesetzten) Zustand des anderen Objekts X durchgeführt werden. Dies kann beispielsweise in einem einzigen Durchlauf des Verfahrens nach Figur 5 wie zeichnerisch angedeutet erfolgen.

## Patentansprüche

1. Medizindatentechnisches Verfahren zum Aktualisieren eines Zustands wenigstens eines Objekts (100, 101, 102, 103, 104, 701, 1000, 1200, 1300, 200, 300, 301, 700, 1400, 1500, 1600, 1700), der eine medizinische Verwendbarkeit des wenigstens einen Objekts (100, 101, 102, 103, 104, 701, 1000, 1200, 1300, 200, 300, 301, 700, 1400, 1500, 1600, 1700) betrifft, das folgende Schritte umfasst:
Bereitstellen (S100, S101, S102) von Objektdaten, die Lagedaten und den Zustand des wenigstens einen Objekts beschreiben;
Bereitstellen von Änderungsdaten (S106), die Änderungen des Zustands des wenigstens einen Objekts (100, 101, 102, 103, 104, 701, 1000, 1200, 1300, 200, 300, 301, 700, 1400, 1500, 1600, 1700) betreffen,
**dadurch gekennzeichnet, dass**
basierend auf einem Vergleich (S104, S108) der Objektdaten mit den Änderungsdaten der Zustand des wenigstens einen Objekts (100, 101, 102, 103, 104, 701, 1000, 1200, 1300, 200, 300, 301, 700, 1400, 1500, 1600, 1700) aktualisiert wird.

2. Verfahren nach Anspruch 1, **dadurch gekennzeichnet, dass** die Änderungsdaten Bedingungen beschreiben, unter denen sich der Zustand des wenigstens einen Objekts (100, 101, 102, 103, 104, 701, 1000, 1200, 1300, 200, 300, 301, 700, 1400, 1500, 1600, 1700) ändert, wobei basierend auf den Änderungsdaten und den Objektdaten bestimmt wird (S106, S104), ob für das wenigstens eine Objekt (100, 101, 102, 103, 104, 701, 1000, 1200, 1300, 200, 300, 301, 700, 1400, 1500, 1600, 1700) eine Bedingung zur Zustandsänderung gegeben ist, und der Zustand, der von den dem wenigstens einen Objekt (100, 101, 102, 103, 104, 701, 1000, 1200, 1300, 200, 300, 301, 700, 1400, 1500, 1600, 1700) zugeordneten Objektdaten beschrieben wird, aktualisiert wird (S109, S110).

3. Verfahren nach einem der vorhergehenden Ansprüche, **dadurch gekennzeichnet, dass** die Änderungsdaten von der Lage wenigstens eines anderen, von dem wenigstens einen Objekt (100, 101, 102, 103, 104, 701, 1000, 1200, 1300, 200, 300, 301, 700, 1400, 1500, 1600, 1700) verschiedenen Objekts (100, 101, 102, 103, 104, 701, 1000, 1200, 1300, 200, 300, 301, 700, 1400, 1500, 1600, 1700) abhängen.

4. Verfahren nach einem der vorhergehenden Ansprüche, **dadurch gekennzeichnet, dass** die Änderungsdaten den Ort des wenigstens einen Objekts (100, 101, 102, 103, 104, 701, 1000, 1200, 1300, 200, 300, 301, 700, 1400, 1500, 1600, 1700) und/oder Zeitinformationen umfassen.

5. Verfahren nach einem der vorhergehenden Ansprüche, **dadurch gekennzeichnet, dass** das Bereitstellen der Objektdaten so abläuft, dass basierend auf den Objektdaten eine Bewegung des wenigstens einen Objekts (100, 101, 102, 103, 104, 701, 1000, 1200, 1300, 200, 300, 301, 700, 1400, 1500, 1600, 1700) nachvollzogen bzw. die zukünftige Bewegung des wenigstens einen Objekts (100, 101, 102, 103, 104, 701, 1000, 1200, 1300, 200, 300, 301, 700, 1400, 1500, 1600, 1700) extrapoliert werden kann.

6. Verfahren nach einem der vorhergehenden Ansprüche, **dadurch gekennzeichnet, dass** die Objektdaten zusätzlich Identifikationsdaten bezüglich des wenigstens einen Objekts (100, 101, 102, 103, 104, 701, 1000, 1200, 1300, 200, 300, 301, 700, 1400, 1500, 1600, 1700) umfassen.

7. Verfahren nach Anspruch 6, **dadurch gekennzeichnet, dass** die Identifikationsdaten auf der Form des wenigstens einen Objekts (100, 101, 102, 103, 104, 701, 1000, 1200, 1300, 200, 300, 301, 700, 1400, 1500, 1600, 1700) und/oder einem Muster des wenigstens einen Objekts (100, 101, 102, 103, 104, 701, 1000, 1200, 1300, 200, 300, 301, 700, 1400, 1500, 1600, 1700) basieren und/oder Daten bezüglich einer Markervorrichtung umfassen.

8. Verfahren nach einem der vorhergehenden Ansprüche, **dadurch gekennzeichnet, dass** basierend auf dem Vergleich (S108, S 104) der Objektdaten mit den Änderungsdaten eine Information über die Aktualisierung des Zustands ausgegeben wird.

9. Verfahren nach einem der vorhergehenden Ansprüche, **dadurch gekennzeichnet, dass** die Objektdaten, wenn sich das wenigstens eine Objekt (100, 101, 102, 103, 104, 701, 1000, 1200, 1300, 200, 300, 301, 700, 1400, 1500, 1600, 1700) wenigstens teilweise in einem Patientenkörper befindet, auf Patientenanalysedaten basieren, die durch ein bildgebendes Verfahren gewonnen werden, um damit die Lage des wenigstens einen Objekts (100, 101, 102, 103, 104, 701, 1000, 1200, 1300, 200, 300, 301, 700, 1400, 1500, 1600, 1700) in einem Patientenkörper zu beschreiben.

10. Verfahren nach einem der vorhergehenden Ansprüche, **dadurch gekennzeichnet, dass** die Objektdaten Soll-Informationen und Ist-Informationen über die Geometrie des wenigstens einen Objekts (100, 101, 102, 103, 104, 701, 1000, 1200, 1300, 200, 300, 301, 700, 1400, 1500, 1600, 1700) beinhalten, wobei die Geometrieinformationen aus beiden Datensätzen miteinander verglichen werden, und basierend auf diesem Vergleich dem wenigstens einen Objekt (100, 101, 102, 103, 104, 701, 1000, 1200, 1300, 200, 300, 301, 700, 1400, 1500, 1600, 1700) ein bestimmter Zustand zugeordnet wird.

11. Verfahren nach einem der vorhergehenden Ansprüche, **dadurch gekennzeichnet, dass** ein Nominalzustand des wenigstens einen Objekts (100, 101, 102, 103, 104, 701, 1000, 1200, 1300, 200, 300, 301, 700, 1400, 1500, 1600, 1700) mit dem Zustand des wenigstens einen Objekts (100, 101, 102, 103, 104, 701, 1000, 1200, 1300, 200, 300, 301, 700, 1400, 1500, 1600, 1700) verglichen wird, worauf basierend auf diesem Vergleich eine Information an eine Verwaltungseinheit gesendet wird.

12. Verfahren nach einem der vorhergehenden Ansprüche, **dadurch gekennzeichnet, dass** die von dem wenigstens einen Objekt (100, 101, 102, 103, 104, 701, 1000, 1200, 1300, 200, 300, 301, 700, 1400, 1500, 1600, 1700) durchlaufenen Zustände protokolliert, d.h. mit dazugehörigen zeitlichen und räumlichen Informationen gespeichert werden und/oder eine bestimmte Abfolge von Zuständen mit einer geplanten Abfolge verglichen werden.

13. Verfahren nach einem der vorhergehenden Ansprüche, **dadurch gekennzeichnet, dass** eine Anfangsanzahl von Objekten (100 ,101, 102, 103, 104, 201, 701, 1200, 1300) und eine Zwischenanzahl und/oder der Endanzahl von Objekten (100, 101, 102, 103, 104, 201, 701, 1200, 1300) ermittelt werden, wobei die Zwischenanzahl und/oder Endanzahl mit der Anfangsanzahl verglichen wird (werden) und basierend auf dem Ergebnis dieses Vergleichs ein Hinweis an einen Benutzer ausgegeben wird.

14. Programm, das wenn es auf einem Computer (1900) läuft oder in einem Computer (1900) geladen ist, den Computer (1900) veranlasst, ein Verfahren gemäß einem der Ansprüche 10 bis 12 auszuführen.

15. Speichermedium (1902), das ein Programm nach Anspruch 14 aufweist oder Signalwelle, die Informationen trägt, die das Programm darstellen.

16. System aus einer Detektionseinrichtung (400, 401) zur Detektion der Lage und Identifikation eines Objekts Objekts (100, 101, 102, 103, 104, 701, 1000, 1200, 1300, 200, 300, 301, 700, 1400, 1500, 1600, 1700) und einer Datenverarbeitungsvorrichtung (1900) zur Ermittlung der Lagedaten und/oder der Identifikationsdaten des Objekts (100, 101, 102, 103, 104, 701, 1000, 1200, 1300, 200, 300, 301, 700, 1400, 1500, 1600, 1700).

## Geänderte Patentansprüche

### Geänderte Patentansprüche gemäss Regel 137(2) EPÜ.

**1.** Medizindatentechnisches Verfahren zum Aktualisieren eines Zustands wenigstens eines Objekts (100, 101, 102, 103, 104, 701, 1000, 1200, 1300, 200, 300, 301, 700, 1400, 1500, 1600, 1700), der eine medizinische Verwendbarkeit des wenigstens einen Objekts (100, 101, 102, 103, 104, 701, 1000, 1200, 1300, 200, 300, 301, 700, 1400, 1500, 1600, 1700) betrifft, das folgende Schritte umfasst:
Erfassen von Lagedaten wenigstens eines Objekts (100, 101, 102, 103, 104, 701, 1000, 1200, 1300, 200, 300, 301, 700, 1400, 1500, 1600, 1700) mittels einer Detektionseinrichtung (400, 401), wobei die Lagedaten eine Beschreibung des Orts des Objekts (100, 101, 102, 103, 104, 701, 1000, 1200, 1300, 200, 300, 301, 700, 1400, 1500, 1600, 1700) umfassen;
Bereitstellen (S100, S101, S102) von Objektdaten, die die Lagedaten und den Zustand des wenigstens einen Objekts (100, 101, 102, 103, 104, 701, 1000, 1200, 1300, 200, 300, 301, 700, 1400, 1500, 1600, 1700) beschreiben;
Bereitstellen von Änderungsdaten (S106), die Änderungen des Zustands des wenigstens einen Objekts (100, 101, 102, 103, 104, 701, 1000, 1200, 1300, 200, 300, 301, 700, 1400, 1500, 1600, 1700) betreffen, wobei die Änderungsdaten den Ort des wenigstens einen Objekts (100, 101, 102, 103, 104, 701, 1000, 1200, 1300, 200, 300, 301, 700, 1400, 1500, 1600, 1700) und/oder Zeitinformationen umfassen;
**dadurch gekennzeichnet, dass**
basierend auf einem Vergleich (S104, S108) der Objektdaten mit den Änderungsdaten der Zustand des wenigstens einen Objekts (100, 101, 102, 103, 104, 701, 1000, 1200, 1300, 200, 300, 301, 700, 1400, 1500, 1600, 1700) aktualisiert wird.

**2.** Verfahren nach Anspruch 1, **dadurch gekennzeichnet, dass** die Änderungsdaten Bedingungen beschreiben, unter denen sich der Zustand des wenigstens einen Objekts (100, 101, 102, 103, 104, 701, 1000, 1200, 1300, 200, 300, 301, 700, 1400, 1500, 1600, 1700) ändert, wobei basierend auf den Änderungsdaten und den Objektdaten bestimmt wird (S106, S104), ob für das wenigstens eine Objekt (100, 101, 102, 103, 104, 701, 1000, 1200, 1300, 200, 300, 301, 700, 1400, 1500, 1600, 1700) eine Bedingung zur Zustandsänderung gegeben ist, und der Zustand, der von den dem wenigstens einen Objekt (100, 101, 102, 103, 104, 701, 1000, 1200, 1300, 200, 300, 301, 700, 1400, 1500, 1600, 1700) zugeordneten Objektdaten beschrieben wird, aktualisiert wird (S109, S110).

**3.** Verfahren nach einem der vorhergehenden Ansprüche, **dadurch gekennzeichnet, dass** die Änderungsdaten von der Lage wenigstens eines anderen, von dem wenigstens einen Objekt (100, 101, 102, 103, 104, 701, 1000, 1200, 1300, 200, 300, 301, 700, 1400, 1500, 1600, 1700) verschiedenen Objekts (100, 101, 102, 103, 104, 701, 1000, 1200, 1300, 200, 300, 301, 700, 1400, 1500, 1600, 1700) abhängen.

**4.** Verfahren nach einem der vorhergehenden Ansprüche, **dadurch gekennzeichnet, dass** das Bereitstellen der Objektdaten so abläuft, dass basierend auf den Objektdaten eine Bewegung des wenigstens einen Objekts (100, 101, 102, 103, 104, 701, 1000, 1200, 1300, 200, 300, 301, 700, 1400, 1500, 1600, 1700) nachvollzogen bzw. die zukünftige Bewegung des wenigstens einen Objekts (100, 101, 102, 103, 104, 701, 1000, 1200, 1300, 200, 300, 301, 700, 1400, 1500, 1600, 1700) extrapoliert werden kann.

**5.** Verfahren nach einem der vorhergehenden Ansprüche, **dadurch gekennzeichnet, dass** die Objektdaten zusätzlich Identifikationsdaten bezüglich des wenigstens einen Objekts (100, 101, 102, 103, 104, 701, 1000, 1200, 1300, 200, 300, 301, 700, 1400, 1500, 1600, 1700) umfassen.

**6.** Verfahren nach Anspruch 5, **dadurch gekennzeichnet, dass** die Identifikationsdaten auf der Form des wenigstens einen Objekts (100, 101, 102, 103, 104, 701, 1000, 1200, 1300, 200, 300, 301, 700, 1400, 1500, 1600, 1700) und/oder einem Muster des wenigstens einen Objekts (100, 101, 102, 103, 104, 701, 1000, 1200, 1300, 200, 300, 301, 700, 1400, 1500, 1600, 1700) basieren und/oder Daten bezüglich einer Markervorrichtung umfassen.

**7.** Verfahren nach einem der vorhergehenden Ansprüche, **dadurch gekennzeichnet, dass** basierend auf dem Vergleich (S108, S104) der Objektdaten mit den Änderungsdaten eine Information über die Aktualisierung des Zustands ausgegeben wird.

**8.** Verfahren nach einem der vorhergehenden Ansprüche, **dadurch gekennzeichnet, dass** die Objektdaten, wenn sich das wenigstens eine Objekt (100, 101, 102, 103, 104, 701, 1000, 1200, 1300, 200, 300, 301, 700, 1400, 1500, 1600, 1700) wenigstens teilweise in einem Patientenkörper befindet, auf Patientenanalysedaten basieren, die durch ein bildgebendes Verfahren gewonnen werden, um damit die Lage des wenigstens einen Objekts (100, 101, 102, 103, 104, 701, 1000, 1200, 1300, 200, 300, 301, 700, 1400, 1500, 1600, 1700) in einem Patientenkörper zu beschreiben.

**9.** Verfahren nach einem der vorhergehenden Ansprüche, **dadurch gekennzeichnet, dass** die Objektdaten Soll-Informationen und Ist-Informationen über die Geometrie des wenigstens einen Objekts (100, 101, 102, 103, 104, 701, 1000, 1200, 1300, 200, 300, 301, 700, 1400, 1500, 1600, 1700) beinhalten, wobei die Geometrieinformationen aus beiden Datensätzen miteinander verglichen werden, und basierend auf diesem Vergleich dem wenigstens einen Objekt (100, 101, 102, 103, 104, 701, 1000, 1200, 1300, 200, 300, 301, 700, 1400, 1500, 1600, 1700) ein bestimmter Zustand zugeordnet wird.

**10.** Verfahren nach einem der vorhergehenden Ansprüche, **dadurch gekennzeichnet, dass** ein Nominalzustand des wenigstens einen Objekts (100, 101, 102, 103, 104, 701, 1000, 1200, 1300, 200, 300, 301, 700, 1400, 1500, 1600, 1700) mit dem Zustand des wenigstens einen Objekts (100, 101, 102, 103, 104, 701, 1000, 1200, 1300, 200, 300, 301, 700, 1400, 1500, 1600, 1700) verglichen wird, worauf basierend auf diesem Vergleich eine Information an eine Verwaltungseinheit gesendet wird.

**11.** Verfahren nach einem der vorhergehenden Ansprüche, **dadurch gekennzeichnet, dass** die von dem wenigstens einen Objekt (100, 101, 102, 103, 104, 701, 1000, 1200, 1300, 200, 300, 301, 700, 1400, 1500, 1600, 1700) durchlaufenen Zustände protokolliert, d.h. mit dazugehörigen zeitlichen und räumlichen Informationen gespeichert werden und/oder eine bestimmte Abfolge von Zuständen mit einer geplanten Abfolge verglichen werden.

**12.** Verfahren nach einem der vorhergehenden Ansprüche, **dadurch gekennzeichnet, dass** eine Anfangsanzahl von Objekten (100 ,101, 102, 103, 104, 201, 701, 1200, 1300) und eine Zwischenanzahl und/oder der Endanzahl von Objekten (100, 101, 102, 103, 104, 201, 701, 1200, 1300) ermittelt werden, wobei die Zwischenanzahl und/oder Endanzahl mit der Anfangsanzahl verglichen wird (werden) und basierend auf dem Ergebnis dieses Vergleichs ein Hinweis an einen Benutzer ausgegeben wird.

**13.** Programm, das wenn es auf einem Computer (1900) läuft oder in einem Computer (1900) geladen ist, den Computer (1900) veranlasst, ein Verfahren gemäß einem der Ansprüche 1 bis 12 auszuführen.

**14.** Speichermedium (1902), das ein Programm nach Anspruch 14 aufweist oder Signalwelle, die Informationen trägt, die das Programm darstellen.

**15.** System aus einer Detektionseinrichtung (400, 401) zur Detektion der Lage und Identifikation eines Objekts (100, 101, 102, 103, 104, 701, 1000, 1200, 1300, 200, 300, 301, 700, 1400, 1500, 1600, 1700) und einer Datenverarbeitungsvorrichtung (1900) zur Ermittlung der Lagedaten und/oder der Identifikationsdaten des Objekts (100, 101, 102, 103, 104, 701, 1000, 1200, 1300, 200, 300, 301, 700, 1400, 1500, 1600, 1700), wobei die Datenverarbeitungsvorrichtung (1900) konfiguriert ist, das Programm nach Anspruch 13 auszuführen.
